# EUROPÄISCHE PATENTSCHRIFT

(11) **EP 3 383 831 B1**
(45) Veröffentlichungstag und Bekanntmachung des Hinweises auf die Patenterteilung: **23.10.2019**
(21) Anmeldenummer: 16805129.0
(22) Anmeldetag: 02.12.2016
(51) Int. Cl.: C07C 5/48

(54) **VERFAHREN ZUR HERSTELLUNG VON BUTADIEN DURCH OXIDATIVE DEHYDRIERUNG VON N-BUTENEN**
METHOD FOR PREPARING BUTADIENE THROUGH THE OXIDATIVE DEHYDRATION OF N-BUTENES
PROCÉDÉ DE FABRICATION DE BUTADIÈNE PAR DÉSHYDRATATION OXYDANTE DE N-BUTÈNES

(30) Priorität: 03.12.2015 EP 15197757
(43) Veröffentlichungstag der Anmeldung: 10.10.2018
(73) Patentinhaber: BASF SE, 67056 Ludwigshafen am Rhein (DE); Linde AG, 80331 München (DE)
(72) Erfinder: JOSCH, Jan Pablo, 67434 Neustadt (DE); BALEGEDDE RAMACHANDRAN, Ragavendra Prasad, 67117 Limburgerhof (DE); GRUENE, Philipp, 68161 Mannheim (DE); RISSEL, Steffen, 67281 Kirchheim (DE); GEMBALA, Martin, New Orleans Louisiana 70115 (US); WENNING, Ulrike, 82049 Pullach (DE); WELLENHOFER, Anton, 82069 Hohenschaeftlarn (DE); REYNEKE, Hendrik, 81479 München (DE); TOEGEL, Christine, 85579 Neubiberg (DE)
(74) Vertreter: Schuck, Alexander
(86) Internationale Anmeldenummer: PCT/EP2016/079534
(87) Internationale Veröffentlichungsnummer: WO 2017/093454

(56) Entgegenhaltungen:
- US-A1- 2012 130 137

## Beschreibung

Die Erfindung betrifft ein Verfahren zur Herstellung von Butadien durch oxidative Dehydrierung von n-Butenen.

Butadien ist eine bedeutende Grundchemikalie und wird beispielsweise zur Herstellung von Synthesekautschuken (Butadien-Homopolymere, Styrol-Butadien-Kautschuk oder NitrilKautschuk) oder zur Herstellung von thermoplastischen Terpolymeren (Acrylnitril-Butadien-Styrol-Copolymere) eingesetzt. Butadien wird ferner zu Sulfolan, Chloropren und 1,4-Hexamethylendiamin (über 1,4-Dichlorbuten und Adipinsäuredinitril) umgesetzt. Durch Dimerisierung von Butadien kann ferner Vinylcyclohexen erzeugt werden, welches zu Styrol dehydriert werden kann.

Butadien kann durch thermische Spaltung (Steam-Cracken) gesättigter Kohlenwasserstoffe hergestellt werden, wobei üblicherweise von Naphtha als Rohstoff ausgegangen wird. Beim Steam-Cracken von Naphtha fällt ein Kohlenwasserstoff-Gemisch aus Methan, Ethan, Ethen, Acetylen, Propan, Propen, Propin, Allen, Butanen, Butenen, Butadien, Butinen, Methylallen, C₅- und höheren Kohlenwasserstoffen an.

Butadien kann auch durch oxidative Dehydrierung von n-Butenen (1-Buten und/oder 2-Buten) erhalten werden. Als Ausgangsgasgemisch für die oxidative Dehydrierung (Oxidehydrierung, ODH) von n-Butenen zu Butadien kann jedes beliebige n-Butene enthaltende Gemisch benutzt werden. Beispielsweise kann eine Fraktion verwendet werden, die als Hauptbestandteil n-Butene (1-Buten und/oder 2-Buten) enthält und aus der C₄-Fraktion eines Naphtha-Crackers durch Abtrennen von Butadien und Isobuten erhalten wurde. Des Weiteren können auch Gasgemische als Ausgangsgas eingesetzt werden, die 1-Buten, cis-2-Buten, trans-2-Buten oder deren Gemische umfassen und durch Dimerisierung von Ethylen erhalten wurden. Ferner können als Ausgangsgas n-Butene enthaltende Gasgemische eingesetzt werden, die durch katalytisches Wirbelschichtcracken (Fluid Catalytic Cracking, FCC) erhalten wurden.

Verfahren zur oxidativen Dehydrierung von Butenen zu Butadien sind grundsätzlich bekannt.

US 2012/0130137A1 beispielsweise beschreibt ein solches Verfahren unter Verwendung von Katalysatoren, die Oxide von Molybdän, Bismuth und in der Regel weiteren Metallen enthalten. Für die nachhaltige Aktivität solcher Katalysatoren für die oxidative Dehydrierung ist ein kritisches Mindestmaß an Sauerstoffpartialdruck in der Gasatmosphäre erforderlich, um eine zu weitgehende Reduktion und damit einen Performanceverlust der Katalysatoren zu vermeiden. Aus diesem Grund kann in der Regel auch nicht mit einem stöchiometrischen Sauerstoffeinsatz oder vollständigem Sauerstoff-Umsatz im Oxidehydrierungsreaktor gearbeitet werden. In der US 2012/0130137 wird zum Beispiel ein Sauerstoffgehalt von 2,5 bis 8 Vol.-% im Ausgangsgas beschrieben.

Die Notwendigkeit eines Sauerstoffüberschusses für solche Katalysatorsysteme ist allgemein bekannt und schlägt sich in den Test- oder Verfahrensbedingungen solcher Katalysatoren nieder. Stellvertretend seien die neueren Arbeiten von Jung et al. (Catal. Surv. Asia 2009, 13, 78-93; DOI 10.1007/s10563-009-9069-5 und Applied Catalysis A: General 2007, 317, 244-249; DOI 10.1016/j.apcata.2006.10.021) genannt.

Bei langen Rohrleitungen (d.h. bei denen die Rohrleitungslänge mindestens ca. 50 mal grösser als der Rohrleitungsdurchmesser ist) kann die Explosion eines Kohlenwasserstoff/Sauerstoff-Gemisches bei entsprechenden stöchiometrischen Verhältnissen (Luftverhältnis Lambda = 1, ideale Mischung) in einer Rohrleitung in eine Detonation umschlagen. Der Umschlag einer Explosion in eine Detonation wird nach Stand dem Technik in der Petrochemie bei gleichzeitiger explosionsfester Bauweise in unter Druck stehenden Systemen z.B. durch folgende Maßnahmen verhindert:
- Vorsehen von Einbauten
- Einbringen von Schüttungen
- Einbau von Explosionsschutzfilter
- Ausführung der Rohrleitungen als Bündel
- Einbau von Löschsystemen (Staub)
- Flüssigkeitsschleier

Derartige Einbauten bringen die Explosion durch eine hohe Masse bei kleinen Spaltweiten und großer Oberfläche zum Erliegen. Die sich ausbreitenden Flamme wird abgekühlt und erlischt im Idealfall schließlich.

Allen diesen Maßnahmen sind kleine Spaltmaße bei großer Oberfläche gemein. Das in Verfahren zur oxidativen Dehydrierung von Butenen zu Butadien durchströmende Kohlenwasserstoff-gemisch enthält jedoch bestimmungsgemäß in nicht unerheblichen Konzentrationen 1,3-Butadien. Dieser Stoff besitzt die Eigenschaft, in Spalten und auf Oberflächen zu polymerisieren. Der Einsatz der genannten Schutzmaßnahmen ist somit in einem Verfahren zur Herstellung von 1,3-Butadien nicht zielführend, da die Betriebszeit bis zur vollständigen Verlegung/Blockage der Einbauten durch Ablagerungen, verursacht durch Polymerisation, zu kurz ist.

Aufgabe der Erfindung ist es, ein Verfahren zur Herstellung von Butadien aus n-Butenen bereitzustellen, das möglichst sicher ist. Ein entsprechendes Sicherheitskonzept muss gewährleisten, dass für Personen und Umwelt keine Gefährdung besteht.

Gelöst wird die Aufgabe durch ein Verfahren zur Herstellung von Butadien aus n-Butenen mit den folgenden Schritten:
A) Bereitstellung eines n-Butene enthaltenden Einsatzgasstroms a;
B) Einspeisung des n-Butene enthaltenden Einsatzgasstromes a und eines sauerstoffhaltigen Gases in mindestens eine Dehydrierzone umfassend einen Dehydrierreaktor und oxidative Dehydrierung von n-Butenen zu Butadien, wobei ein Produktgasstrom b enthaltend Butadien, nicht umgesetzte n-Butene, Wasserdampf, Sauerstoff, leicht siedende Kohlenwasserstoffe, gegebenenfalls Kohlenstoffoxide und gegebenenfalls Inertgase erhalten wird;
C) Abkühlung und Kompression des Produktgasstroms b in mindestens einer Abkühlstufe umfassend mindestens eine Quenchkolonne und in mindestens einer Kompressionsstufe umfassend mindestens einen Kompressor und gegebenenfalls einen oder mehrere Zwischenkühler zwischen den Kompressoren, wobei der Produktgasstrom b mit mindestens einem im Kreis geführtem Kühlmittel in Kontakt gebracht wird, wobei mindestens ein Kondensatstrom c1 enthaltend Wasser und ein Gasstrom c2 enthaltend Butadien, n-Butene, Wasserdampf, Sauerstoff, leicht siedende Kohlenwasserstoffe, gegebenenfalls Kohlenstoffoxide und gegebenenfalls Inertgase erhalten wird;
D) Abtrennung von nicht kondensierbaren und leicht siedenden Gasbestandteilen umfassend Sauerstoff, leicht siedende Kohlenwasserstoffe, gegebenenfalls Kohlenstoffoxide und gegebenenfalls Inertgase als Gasstrom d2 aus dem Gasstrom c2 durch Absorption der C₄-Kohlenwasserstoffe umfassend Butadien und n-Butene in mindestens einem im Kreis geführten Absorptionsmittel, wobei mindestens ein mit C₄-Kohlenwasserstoffen beladener Absorptionsmittelstrom und der Gasstrom d2 erhalten werden, und anschließende Desorption der C₄-Kohlenwasserstoffe aus dem beladenen Absorptionsmittelstrom, wobei ein C₄-Produktgasstrom d1 erhalten wird;
E) Auftrennung des C₄-Produktstroms d1 durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2;
F) Destillation des Butadien und das selektive Lösungsmittel enthaltenden Stoffstroms e1 in einen im Wesentlichen aus dem selektiven Lösungsmittel bestehenden Stoffstrom f1 und einen Butadien enthaltenden Stoffstrom f2;
dadurch gekennzeichnet, dass die nachstehenden Maßnahmen (i) bis (iii) durchgeführt werden:
(i) Vermeidung der Bildung explosionsfähiger Gasgemische durch Überwachung der Sauerstoffkonzentration in den in die Dehydrierzone eingespeisten sauerstoffhaltigen Gasströmen und Steuerung der Massenströme von sauerstoffhaltigen Gasströmen und Kohlenwasserstoffe enthaltenden Gasströmen derart, dass sich keine explosionsfähigen Gasgemische ausbilden können;
(ii) Unterbrechung der Zufuhr des sauerstoffhaltigen Gasgemischs in die Dehydrierzone bei Überschreitung eines Grenzwertes für die Sauerstoffkonzentration in dem Dehydriergasgemisch;
(iii) Durchführung der Schritte A) bis F) in Apparaten, die explosionsfest ausgeführt sind, wobei flüssigkeitsführende Rohrleitungen explosionsfest und Gasleitungen detonationsfest ausgeführt sind.

Erfindungsgemäß wird die Sicherheit des Verfahrens durch ein Bündel von Maßnahmen gewährleistet. Zum einen wird eine sicherheitsgerichtete Abschaltung der Anlage zum Erhalt der inneren Integrität der Anlage (Einbauten) vorgesehen. So erfolgt durch geeignete online-Analytik eine Überwachung der unter Umständen schwankenden Sauerstoffkonzentration in den sauerstoffhaltigen Gasströmen, die in den Dehydrierreaktor eingespeist werden. Weiterhin erfolgt eine Überwachung der Massenströme von sauerstoffhaltigen Gasströmen und Kohlenwasserstoff-haltigen Gasströmen durch zertifizierte Mengenmessungen mit entsprechenden Verhältnisregelungen. Dadurch wird die Einhaltung eines Mindestabstands der aktuellen Sauerstoffkonzentration in den Verfahrensströmen von der unteren Explosionsgrenze unter Berücksichtigung von Betriebsschwankungen und der Genauigkeit der online-Analytik sichergestellt. Bei Überschreitung der Grenzwerte der Sauerstoffkonzentration an der unteren Explosiongrenze erfolgt eine Abschaltung des Reaktors oder der gesamten Anlage.

Unter "Abschalten des Reaktors" versteht man die Unterbrechung der Feedgasströme zum Reaktor, jedoch mindestens die Unterbrechung der Sauerstoffzuführung zum Reaktor, z.B. durch Schließen von Ventilen in den betreffenden Rohrleitungen.

Unter "Abschalten der gesamten Anlage" versteht man die Unterbrechung von allen relevanten Feedgasströmen in die Anlage, von Produktströmem aus der Anlage sowie von Strömen innerhalb der Anlage z.B. durch Schließen von Ventilen in den betreffenden Rohrleitungen sowie gegebenefalls die Durchführung weiterer Maßnahmen zur Überführung der Anlage in einen sicheren Zustand.

Zum anderen werden gasführende Apparate in den von Explosionsgefahr betroffenen Baugruppen explosionsfest ausgeführt und gasführende Rohrleitungen detonationsfest ausgeführt, um die Integrität der Anlage zur Umgebung hin sicherzustellen. So werden gasführende Apparate und flüssigkeitsführende Rohrleitungen in den betroffenen Baugruppen explosionsfest ausgeführt. Im Gegensatz zum Stand der Technik werden gasführende Rohrleitungen, deren unverbaute Länge mindestens das 50-fache ihres Durchmessers beträgt, detonationsfest ausgelegt. Die Kombination der explosionsfesten Auslegung von gasdurchströmten, im Sinne einer kleinen Anlauflänge "kurzen" Apparate und der detonationfesten Auslegung von "langen" Rohrleitungen führt zu einem inhärent sicheren Anlagendesign. Längen und Durchmesser der gasdurchströmten Apparate werden so gewählt, dass eine Detonation ausgeschlossen werden kann. Zudem werden Einbauten (Böden) in Destillationskolonnen gasdicht ausgeführt. Gaszuleitungen werden, falls prozesstechnisch möglich, in die Flüssigkeit abgetaucht, um ein Durchzünden einer Explosion in der Rohrleitung in die verbundenen Anlagenteile zu vermeiden. Kühler werden vorzugsweise statt als Wärmetauscher als Direktkühler ausgeführt.

Vorzugsweise sind einer oder mehrere der nachstehenden Apparate explosionsfest ausgelegt:
- der in Schritt B) eingesetzte Dehydrierreaktor;
- die in Schritt C) eingesetzte Quenchkolonne;
- die in Schritt C) eingesetzten Kompressoren;
- die in Schritt C) eingesetzten Zwischenkühler;
- die in Schritt F) eingesetzte Destillationskolonne;
- der in der Lösungsmittelregeneriereinheit eingesetzte Dekanter.

Zur explosionsfesten Ausführung wird folgendes ausgeführt: Bei einer Explosion handelt es sich um einen unkontrollierten Abbrand eines zündfähigen Gemisches mit laminarer Flammfront. Für stöchiometrische Luft / Kohlenwasserstoffgemische wird ein maximaler Explosionsdruckanstieg um den Faktor 10 errechnet.

Somit bedeutet explosionsfeste Ausführung, dass der Apparat bzw. die Rohrleitung für einen Druckanstieg um bis zu einem Faktor 10 ausgelegt ist.

Zur detonationsfesten Ausführung wird folgendes ausgeführt: Eine Explosion kann nach einer Anlauflänge und einer Mindestkonzentration an Brennstoff und Sauerstoff in eine Detonation umschlagen. Das passiert dann, wenn sich die Flammenfront mit mehr als Schallgeschwindigkeit beschleunigt. Der Druckanstiegsfaktor kann 50 und mehr betragen.

Durch geeignete Wahl des Verhältnisses Länge : Durchmesser einer gasführenden Rohrleitung und eines Apparates kann einen Detonation ausgeschlossen werden. Dieses Verhältnis darf im Allgemeinen höchstens 50 : 1 betragen. Beträgt das Verhältnis mehr als 50 : 1, wird die Rohrleitung detonationsfest ausgelegt.

Somit weisen erfindungsgemäß zwischen den Apparaten der Stufen A) bis F) Gasleitungen entweder ein derartiges Verhältnis von Länge zu Durchmesser auf, so dass eine Detonation ausgeschlossen ist, oder die betreffenden gasführenden Rohrleitungen werden detonationsfest ausgelegt. Dieses Verhältnis beträgt im Allgemeinen höchstens 50 : 1. Bei einem größeren Verhältnis werden die Leitungen detonationsfest ausgelegt. Betroffene Rohrleitungen werden beispielhaft für das 50-fache des maximalen Betriebsdrucks ausgelegt. Bei einem Betriebsdruck von 5 bar errechnet sich beispielhaft ein Auslegungsdruck von 250 bar. Der Werkstoff und die Wandstärke der Rohrleitung sind dann entsprechend diesem Druck auszuwählen (= mechanische Auslegung).

Die in Schritt C) eingesetzten Zwischenkühler sind vorzugsweise als Direktkühler ausgeführt. Diese Direktkühler werden als vertikale Kolonnen mit Einbauten ausgeführt. Als Einbauten kommen Schüttungen wie z.B. Pallringe, Packungen oder vorzugsweise Kolonnenböden wie z.B. Siebböden, Kaskadenböden oder Ventilböden in Frage. Ein flüssiges Kühlmedium wie zum Beispiel Wasser oder ein Kohlenwasserstoff wie Toluol, bevorzugt Mesitylen wird im Gleichstrom oder bevorzugt im Gegenstrom zum zu kühlenden Gasstrom geführt.

Die in den Schritten D), E) und/oder F) eingesetzten Destillationskolonnen sind vorzugsweise als Bodenkolonnen mit gasdichten Kolonnenböden ausgeführt.

Weiterhin bevorzugt ist es, dass Gaszuleitungen in die in den Schritten D), E) und/oder F) eingesetzten Destillationskolonnen in die Flüssigkeit eintauchen.

Gasleitungen zwischen den Apparaten der Stufen A) bis F) weisen ein derartiges Verhältnis Länge : Durchmesser auf, dass eine Detonation unmöglich ist. Vorzugsweise beträgt das Verhältnis Länge : Durchmesser höchstens 50 : 1.

Als Kühlmittel können in Schritt C) Wasser, alkalisch-wässrige Lösungen, organische Lösungsmittel oder Gemische daraus verwendet werden. Vorzugsweise wird ein organisches Lösungsmittel verwendet. Bevorzugt sind aromatische Kohlenwasserstoff-Lösungsmittel wie Toluol, Xylol oder Mesitylen.

Nachstehende Ausführungsformen sind bevorzugte bzw. besonders bevorzugte Varianten des erfindungsgemäßen Verfahrens:
Die Stufe C) umfasst mindestens eine Abkühlungsstufe Ca) und eine Kompressionsstufe Cb). Die Stufe Ca) wird bevorzugt mehrstufig in Stufen Ca1) bis Can), insbesondere zweistufig in zwei Stufen Ca1) und Ca2) durchgeführt. Dabei wird in einer Variante zumindest ein Teil des Kühlmittels nach Durchlaufen der zweiten Stufe Ca2) als Kühlmittel der ersten Stufe Ca1) zugeführt.

Die Stufe Cb) umfasst im Allgemeinen mindestens eine Kompressionsstufe Cba) und mindestens eine Abkühlstufe Cbb). Bevorzugt wird in der mindestens einen Abkühlstufe Cbb) das in der Kompressionsstufe Cba) komprimierte Gas mit einem Kühlmittel in Kontakt gebracht. Besonders bevorzugt enthält das Kühlmittel der Abkühlstufe Cbb) das gleiche organische Lösungsmittel, das in der Stufe Ca) als Kühlmittel verwendet wird. In einer insbesonders bevorzugten Variante wird zumindest ein Teil dieses Kühlmittels nach Durchlaufen der mindestens einen Abkühlstufe Cbb) als Kühlmittel der Stufe Ca) zugeführt.

Bevorzugt umfasst die Stufe Cb) mehrere Kompressionsstufen Cba1) bis Cban) und Abkühlstufen Cbb1) bis Cbbn), beispielsweise vier Kompressionsstufen Cba1) bis Cba4) und vier Abkühlstufen Cbb1) bis Cbb4).

Die Kompressionsstufen Cba1) bis Cban) sind vorzugsweise alle explosionsfest ausgelegt.

Die Abkühlstufen Cbb1) bis Cbbn) sind vorzugsweise alle als Direktkühler ausgebildet.

Bevorzugt umfasst Schritt D) die Schritte Da) bis Dc):
Da) Absorption der C₄-Kohlenwasserstoffe umfassend Butadien und n-Butene in einem hochsiedenden Absorptionsmittel, wobei ein mit C₄-Kohlenwasserstoffen beladener Absorptionsmittelstrom und der Gasstrom d2 erhalten werden,
Db) Entfernung von Sauerstoff aus dem mit C₄-Kohlenwasserstoffen beladenen Absorptionsmittelstrom aus Schritt Da) durch Strippung mit einem nicht kondensierbaren Gasstrom, und
Dc) Desorption der C₄-Kohlenwasserstoffe aus dem beladenen Absorptionsmittelstrom, wobei ein C₄-Produktgasstrom d1 erhalten wird. Dieser weist dann bevorzugt einen Sauerstoffgehalt von weniger als 100 ppm auf.

In einer Ausführungsform ist das in Schritt D) verwendete Absorptionsmittel das gleiche organische Lösungsmittel wie ein in Schritt C) verwendetes Kühlmittel, wobei zumindest ein Teil dieses Absorptionsmittels nach Desorption der C₄-Kohlenwasserstoffe als Kühlmittel dem Schritt C) zugeführt wird. In einer bevorzugten Variante dieser Ausführungsform ist das Absorptions- und Kühlmittel Toluol, Xylol oder Mesitylen.

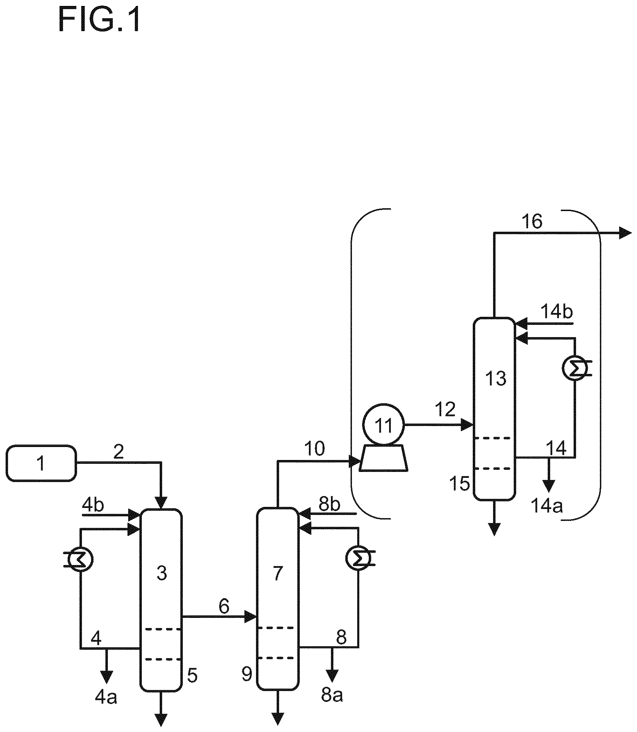

Bevorzugte Ausführungsformen des Verfahrens sind in den Figuren 1 bis 3 dargestellt und werden im Folgenden im Einzelnen beschrieben.

In einem Schritt A) wird ein n-Butene enthaltender Einsatzgasstrom bereitgestellt.

Als Einsatzgasstrom können reine n-Butene (1-Buten und/oder cis-/trans-2-Buten), aber auch Butene enthaltende Gasgemische eingesetzt werden. Ein solches Gasgemisch kann beispielsweise durch nicht-oxidative Dehydrierung von n-Butan erhalten werden. Es kann auch eine Fraktion eingesetzt werden, die als Hauptbestandteil n-Butene (1-Buten und cis-/trans-2-Buten) enthält und aus der C₄-Fraktion des Naphtha-Crackens durch Abtrennung von Butadien und iso-Buten erhalten wurde. Des Weiteren können auch Gasgemische als Ausgangsgas eingesetzt werden, die reines 1-Buten, cis-2-Buten, trans-2-Buten oder Mischungen daraus umfassen, und die durch Dimerisierung von Ethylen erhalten wurden. Ferner können als Ausgangsgas n-Butene enthaltende Gasgemische eingesetzt werden, die durch katalytisches Wirbelschichtkracken (Fluid Catalytic Cracking, FCC) erhalten wurden.

In einer Ausführungsform des erfindungsgemäßen Verfahrens wird das n-Butene enthaltende Ausgangsgasgemisch durch nicht-oxidative Dehydrierung von n-Butan erhalten. Durch die Kopplung einer nicht-oxidativen katalytischen Dehydrierung mit der oxidativen Dehydrierung der gebildeten n-Butene kann eine hohe Ausbeute an Butadien, bezogen auf eingesetztes n-Butan, erhalten werden. Bei der nicht-oxidativen katalytischen n-Butan-Dehydrierung wird ein Gasgemisch erhalten, das neben Butadien 1-Buten, 2-Buten und nicht umgesetztem n-Butan Nebenbestandteile enthält. Übliche Nebenbestandteile sind Wasserstoff, Wasserdampf, Stickstoff, CO und CO₂, Methan, Ethan, Ethen, Propan und Propen. Die Zusammensetzung des die erste Dehydrierzone verlassenden Gasgemischs kann abhängig von der Fahrweise der Dehydrierung stark variieren. So weist bei Durchführung der Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

In Schritt B) werden der n-Butene enthaltende Einsatzgasstrom und ein sauerstoffhaltiges Gas in mindestens eine Dehydrierzone 1 (ODH-Reaktor) eingespeist und die in dem Gasgemisch enthaltenen Butene in Gegenwart eines Oxidehydrierungskatalysators oxidativ zu Butadien dehydriert.

Für die Oxidehydrierung geeignete Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten, wie beispielsweise Kalium, Cäsium, Magnesium, Zirkon, Chrom, Nickel, Cobalt, Cadmium, Zinn, Blei, Germanium, Lanthan, Mangan, Wolfram, Phosphor, Cer, Aluminium oder Silizium. Auch eisenhaltige Ferrite wurden als Katalysatoren vorgeschlagen.

In einer bevorzugten Ausführungsform enthält das Multimetalloxid Cobalt und/oder Nickel. In einer weiteren bevorzugten Ausführungsform enthält das Multimetalloxid Chrom. In einer weiteren bevorzugten Ausführungsform enthält das Multimetalloxid Mangan.

Beispiele für Mo-Bi-Fe-O-haltige Multimetalloxide sind Mo-Bi-Fe-Cr-O- oder Mo-Bi-Fe-Zr-O-haltige Multimetalloxide. Bevorzugte Systeme sind beispielsweise beschrieben in US 4,547,615 (Mo₁₂BiFe_{0,1}Ni₈ZrCr₃K_{0,2}Oₓ und Mo₁₂BiFe_{0,1}Ni₈AlCr₃K_{0,2}Oₓ), US 4,424,141 (Mo₁₂BiFe₃Co_{4,5}Ni₂,₅P_{0,5}K_{0,1}Oₓ + SiO₂), DE-A25 30 959 (Mo₁₂BiFe₃Co₄,₅Ni₂,₅Cr_{0,5}K_{0,1}Oₓ, Mo_{13,75}BiFe₃Co_{4,5}Ni_{2,5}Ge_{0,5}K₀,₈Oₓ, Mo₁₂BiFe₃Co_{4,5}Ni₂,₅Mn_{0,5}K_{0,1}Oₓ und Mo₁₂BiFe₃Co_{4,5}Ni_{2,5}La_{0,5}K_{0,1}Oₓ), US 3,911,039 (Mo₁₂BiFe₃Co_{4,5}Ni₂,₅Sn_{0,5}K_{0,1}Oₓ), DE-A25 30 959 und DE-A24 47 825 (Mo₁₂BiFe₃Co_{4,5}Ni_{2,5}W_{0,5}K_{0,1}Oₓ).

Geeignete Multimetalloxide und deren Herstellung sind weiterhin beschrieben in US 4,423,281 (Mo₁₂BiNi₈Pb_{0,5}Cr₃K_{0,2}Ox und Mo₁₂Bi_{b}Ni₇Al₃Cr_{0,5}K_{0,5}Oₓ), US 4,336,409 (Mo₁₂BiNi₆Cd₂Cr₃P_{0,5}Oₓ), DE-A 26 00 128 (Mo₁₂BiNi_{0,5}Cr₃P_{0,5}Mg_{7,5}K_{0,1}Oₓ + SiO₂) und DE-A 24 40 329 (Mo₁₂BiCo_{4,5}Ni_{2,5}Cr₃P_{0,5}K_{0,1}Oₓ).

Besonders bevorzugte katalytisch aktive, Molybdän und mindestens ein weiteres Metall enthaltende Multimetalloxide weisen die allgemeine Formel (Ia) auf:

Mo₁₂BiₐFe_{b}Co_{c}Ni_{d}CrₑX¹_{f}X²_{g}O_{y} (Ia),

mit
X¹ = Si, Mn und/oder Al,
X² = Li, Na, K, Cs und/oder Rb,
0,2 ≤ a ≤ 1,
0,5 ≤ b ≤ 10,
0 ≤ c ≤ 10,
0 ≤ d ≤ 10,
2 ≤ c + d ≤ 10
0 ≤ e ≤ 2,
0 ≤ f ≤ 10
0 ≤ g ≤ 0,5
y = eine Zahl, die unter der Voraussetzung der Ladungsneutralität durch die Wertigkeit und Häufigkeit der von Sauerstoff verschiedenen Elemente in (la) bestimmt wird.

Bevorzugt sind Katalysatoren, deren katalytisch aktive Oxidmasse von den beiden Metallen Co und Ni nur Co aufweist (d = 0). Bevorzugte ist X¹ Si und/oder Mn und X² ist vorzugsweise K, Na und/oder Cs, besonders bevorzugt ist X² = K.

Das molekularen Sauerstoff enthaltende Gas enthält im Allgemeinen mehr als 10 Vol.-%, vorzugsweise mehr als 15 Vol.-% und noch mehr bevorzugt mehr als 20 Vol.-% molekularen Sauerstoff. Bevorzugt ist es Luft. Die Obergrenze für den Gehalt an molekularem Sauerstoff beträgt im Allgemeinen 50 Vol.-% oder weniger, vorzugsweise 30 Vol.-% oder weniger und noch mehr bevorzugt 25 Vol.-% oder weniger. Darüber hinaus können in dem molekularen Sauerstoff enthaltenden Gas beliebige Inertgase enthalten sein. Als mögliche Inertgase können Stickstoff, Argon, Neon, Helium, CO, CO₂ und Wasser genannt werden. Die Menge an Inertgasen beträgt für Stickstoff im Allgemeinen 90 Vol.-% oder weniger, vorzugsweise 85 Vol.-% oder weniger und noch mehr bevorzugt 80 Vol.-% oder weniger. Im Falle anderer Bestandteile als Stickstoff beträgt sie im Allgemeinen 10 Vol.-% oder weniger, vorzugsweise 1 Vol.-% oder weniger.

Zur Durchführung der oxidativen Dehydrierung bei Vollumsatz von n-Butenen ist ein Gasgemisch bevorzugt, welches ein molares Sauerstoff: n-Butene-Verhältnis von mindestens 0,5 aufweist. Bevorzugt wird bei einem Sauerstoff: n-Butene-Verhältnis von 0,55 bis 10 gearbeitet. Zur Einstellung dieses Wertes kann das Ausgangsstoffgas mit Sauerstoff oder einem sauerstoffhaltigem Gas, beispielsweise Luft, und gegebenenfalls zusätzlichem Inertgas oder Wasserdampf vermischt werden. Das erhaltene sauerstoffhaltige Gasgemisch wird dann der Oxidehydrierung zugeführt.

Die Reaktionstemperatur der Oxidehydrierung wird im Allgemeinen durch ein Wärmeaustauschmittel, welches sich um die Reaktionsrohre herum befindet, kontrolliert. Als solche flüssige Wärmeaustauschmittel kommen z.B. Schmelzen von Salzen wie Kaliumnitrat, Kaliumnitrit, Natriumnitrit und/oder Natriumnitrat sowie Schmelzen von Metallen wie Natrium, Quecksilber und Legierungen verschiedener Metalle in Betracht. Aber auch ionische Flüssigkeiten oder Wärmeträgeröle sind einsetzbar. Die Temperatur des Wärmeaustauschmittels liegt zwischen 220 bis 490 °C und bevorzugt zwischen 300 bis 450 °C und besonders bevorzugt zwischen 350 und 420 °C.

Auf Grund der Exothermie der ablaufenden Reaktionen kann die Temperatur in bestimmten Abschnitten des Reaktorinneren während der Reaktion höher liegen als diejenige des Wärmeaustauschmittels und es bildet sich ein so genannter Hotspot aus. Die Lage und Höhe des Hotspots ist durch die Reaktionsbedingungen festgelegt, aber sie kann auch durch das Verdünnungsverhältnis der Katalysatorschicht oder den Durchfluss an Mischgas reguliert werden. Die Differenz zwischen Hotspot-Temperatur und der Temperatur des Wärmeaustauschmittels liegt im Allgemeinen zwischen 1 und 150 °C, bevorzugt zwischen 10 und 100 °C und besonders bevorzugt zwischen 20 und 80 °C. Die Temperatur am Ende des Katalysatorbettes liegt im Allgemeinen zwischen 0 und 100 °C, vorzugsweise zwischen 0,1 und 50 °C, besonders bevorzugt zwischen 1 und 25 °C oberhalb der Temperatur des Wärmeaustauschmittels.

Die Oxidehydrierung kann in allen aus dem Stand der Technik bekannten Festbettreaktoren durchgeführt werden, wie beispielsweise im Hordenofen, im Festbettrohr- oder Rohrbündelreaktor oder im Plattenwärmetauscherreaktor. Ein Rohrbündelreaktor ist bevorzugt.

Der Reaktor ist explosionsfest ausgelegt. Hierbei wird der Mantel des Reaktors entsprechend dem zu erwartenden Explosionsdruck ausgelegt. Beispielsweise beträgt der maximale Betriebsdruck des Reaktors 5 bar, der Explosionsdruckfaktor des im Reaktor befindlichen Gasgemisches beträgt 10. Damit ergibt sich für die mechanische Auslegung des Reaktormantels ein Designdruck von 50 bar. Werkstoff sowie Wandstärke werden dann gemäß dem Designdruck von 50 bar gewählt.

Vorzugsweise wird die oxidative Dehydrierung in Festbettrohrreaktoren oder Festbettrohrbündelreaktoren durchgeführt. Die Reaktionsrohre werden (ebenso wie die anderen Elemente des Rohrbündelreaktors) in der Regel aus Stahl gefertigt. Die Wanddicke der Reaktionsrohre beträgt typischerweise 1 bis 3 mm. Ihr Innendurchmesser liegt in der Regel (einheitlich) bei 10 bis 50 mm oder bei 15 bis 40 mm, häufig bei 20 bis 30 mm. Die im Rohrbündelreaktor untergebrachte Anzahl an Reaktionsrohren beläuft sich in der Regel wenigstens auf 1000, oder 3000, oder 5000, vorzugsweise auf wenigstens 10 000. Häufig beträgt die Anzahl der im Rohrbündelreaktor untergebrachten Reaktionsrohre 15 000 bis 30 000 bzw. bis 40 000 oder bis 50 000. Die Länge der Reaktionsrohre erstreckt sich im Normalfall auf wenige Meter, typisch ist eine Reaktionsrohrlänge im Bereich von 1 bis 8 m, häufig 2 bis 7 m, vielfach 2,5 bis 6 m.

Weiterhin kann die Katalysatorschicht, die im Reaktor 1 eingerichtet ist, aus einer einzelnen Schicht oder aus 2 oder mehr Schichten bestehen. Diese Schichten können aus reinem Katalysator bestehen oder mit einem Material verdünnt sein, das nicht mit dem Ausgangsgas oder Komponenten aus dem Produktgas der Reaktion reagiert. Weiterhin können die Katalysatorschichten aus Vollmaterial oder geträgerten Schalenkatalysatoren bestehen.

Der die oxidative Dehydrierung verlassende Produktgasstrom 2 enthält neben Butadien im Allgemeinen noch nicht umgesetztes 1-Buten und 2-Buten, Sauerstoff sowie Wasserdampf. Als Nebenkomponenten enthält er weiterhin im Allgemeinen Kohlenmonoxid, Kohlendioxid, Inertgase (hauptsächlich Stickstoff), leichtsiedende Kohlenwasserstoffe wie Methan, Ethan, Ethen, Propan und Propen, Butan und iso-Butan, gegebenenfalls Wasserstoff sowie gegebenenfalls sauerstoffhaltige Kohlenwasserstoffe, sogenannte Oxygenate. Oxygenate können beispielsweise Formaldehyd, Furan, Essigsäure, Maleinsäureanhydrid, Ameisensäure, Methacrolein, Methacrylsäure, Crotonaldehyd, Crotonsäure, Propionsäure, Acrylsäure, Methylvinylketon, Styrol, Benzaldehyd, Benzoesäure, Phthalsäureanhydrid, Fluorenon, Anthrachinon und Butyraldehyd sein.

Der Produktgasstrom 2 am Reaktorausgang ist durch eine Temperatur nahe der Temperatur am Ende des Katalysatorbetts charakterisiert. Der Produktgasstrom wird dann auf eine Temperatur von 150 bis 400 °C, bevorzugt 160 bis 300 °C, besonders bevorzugt 170 bis 250 °C gebracht. Es ist möglich, die Leitung, durch die der Produktgasstrom fließt, um die Temperatur im gewünschten Bereich zu halten, zu isolieren, jedoch ist ein Einsatz eines Wärmetauschers bevorzugt. Dieses Wärmetauschersystem ist beliebig, solange mit diesem System die Temperatur des Produktgases auf dem gewünschten Niveau gehalten werden kann. Als Beispiel eines Wärmetauschers können Spiralwärmetauscher, Plattenwärmetauscher, Doppelrohrwärmetauscher, Multirohrwärmetauscher, Kessel-Spiralwärmetauscher, Kessel-Mantelwärmetauscher, Flüssigkeit-Flüssigkeit-Kontakt-Wärmetauscher, Luft-Wärmetauscher, Direktkontaktwärmetauscher sowie Rippenrohrwärmetauscher genannt werden. Da, während die Temperatur des Produktgases auf die gewünschte Temperatur eingestellt wird, ein Teil der hochsiedenden Nebenprodukte, die im Produktgas enthalten sind, ausfallen kann, sollte daher das Wärmetauschersystem vorzugsweise zwei oder mehr Wärmetauscher aufweisen. Falls dabei zwei oder mehr vorgesehene Wärmetauscher parallel angeordnet sind, und so eine verteilte Kühlung des gewonnenen Produktgases in den Wärmetauschern ermöglicht wird, nimmt die Menge an hochsiedenden Nebenprodukten, die sich in den Wärmetauschern ablagern, ab und so kann ihre Betriebsdauer verlängert werden. Als Alternative zu der oben genannten Methode können die zwei oder mehr vorgesehenen Wärmetauscher parallel angeordnet sein. Das Produktgas wird einem oder mehreren, nicht aber allen, Wärmetauschern zugeführt, welche nach einer gewissen Betriebsdauer von anderen Wärmetauschern abgelöst werden. Bei dieser Methode kann die Kühlung fortgesetzt werden, ein Teil der Reaktionswärme zurückgewonnen und parallel dazu können die in einem der Wärmetauscher abgelagerten hochsiedenden Nebenprodukte entfernt werden. Als ein oben genanntes organisches Lösungsmittel kann ein Lösungsmittel, solange es in der Lage ist, die hochsiedenden Nebenprodukte aufzulösen, uneingeschränkt verwendet werden, und als Beispiele dazu können ein aromatisches Kohlenwasserstofflösungsmittel, wie z.B. Toluol, Xylen etc. sowie ein alkalisches wässriges Lösungsmittel, wie z.B. die wässrige Lösung von Natriumhydroxid, verwendet werden.

Anschließend können im Schritt C) aus dem Produktgasstrom 2 durch Abkühlung ein Großteil der hochsiedenden Nebenkomponenten und des Wassers abgetrennt werden. Diese Abkühlung und Abtrennung erfolgt dabei vorzugsweise in einem Quench. Dieser Quench kann aus einer (3 in Figur 1) oder mehreren Stufen bestehen (3, 7 in Figur 1). Vorzugsweise wird ein Verfahren eingesetzt, bei dem der Produktgasstrom 2 direkt mit dem Kühlmittel 4 in Kontakt gebracht und dadurch gekühlt wird. Als Kühlmittel können Wasser, alkalische wässrige Lösungen, organische Lösungsmittel oder eine Kombination oder Gemisch daraus verwendet werden. Vorzugsweise wird ein organisches Lösungsmittel verwendet. Als Lösungsmittel kann, solange es in der Lage ist, Teile der im Gasstrom befindlichen Nebenkomponenten aufzunehmen, jedes Lösungsmittel verwendet werden. Besonders bevorzugt sind Lösungsmittel wie Toluol, Xylol oder Mesitylen.

In einer bevorzugten Ausführungsform des erfindungsgemäßen Verfahrens wird Toluol als Kühlmittel verwendet. In einer besonders bevorzugten Ausführungsform des erfindungsgemäßen Verfahrens wird Mesitylen als Kühlmittel verwendet.

Bevorzugt ist ein zweistufiger Quench. Die Kühlungstemperatur des Produktgases unterscheidet sich je nach Art der Temperatur des aus dem Reaktorauslass erhaltenen Produktgases 2 und des Kühlmittels 4. Im Allgemeinen hat das Produktgas 2 je nach Vorliegen und Temperaturniveau eines Wärmetauschers vor dem Quencheingang eine Temperatur von 100 bis 440 °C. Der Produktgaseinlass in den Quench muss so ausgelegt sein, dass ein Verstopfen durch Ablagerungen am und direkt vor dem Gaseinlass minimiert oder verhindert wird. Das Produktgas wird in der 1. Quenchstufe 3 mit dem Kühlmittel in Kontakt gebracht. Hierbei kann das Kühlmittel durch eine Düse eingebracht werden, um eine möglichst effiziente Durchmischung mit dem Produktgas zu erreichen. Zum gleichen Zweck können in der Quenchstufe Einbauten, wie zum Beispiel weitere Düsen, eingebracht werden, durch die das Produktgas und das Kühlmittel gemeinsam passieren müssen. Der Kühlmitteleinlass in den Quench muss so ausgelegt sein, dass ein Verstopfen durch Ablagerungen im Bereich des Kühlmitteleinlasses minimiert oder verhindert wird.

Im Allgemeinen wird das Produktgas 2 in der ersten Quenchstufe auf 5 bis 180 °C, vorzugsweise auf 30 bis 130 °C und noch mehr bevorzugt auf 60 bis 110 °C gekühlt. Die Temperatur des Kühlmittelmediums 4 am Einlass kann im Allgemeinen 25 bis 200 °C, bevorzugt 40 bis 120 °C, insbesondere bevorzugt 50 bis 90 °C betragen. Der Druck in der ersten Quenchstufe ist nicht besonders eingeschränkt, beträgt aber im Allgemeinen 0,01 bis 4 bar (ü), bevorzugt 0,1 bis 2 bar (ü) und besonders bevorzugt 0,2 bis 1 bar (ü). Wenn viel hochsiedende Nebenprodukte im Produktgas vorhanden sind, kommt es leicht zu Polymerisation der hochsiedenden Nebenprodukte und zu Ablagerungen von Feststoffen, die durch hochsiedende Nebenprodukte in diesem Verfahrensabschnitt verursacht werden. Das im Kühlturm der ersten Quenchstufe eingesetzt Kühlmittel 4 wird zirkulierend eingesetzt. Der Kreislaufstrom des Kühlmittels in Liter pro Stunde bezogen auf den Massenstrom an Butadien in Gramm pro Stunde kann im Allgemeinen 0,0001 bis 5 l/g, bevorzugt 0,001 bis 1 l/g und besonders bevorzugt 0,002-0,2 l/g betragen.

Die Temperatur des Kühlmittels 4 im Sumpf kann im Allgemeinen 27 bis 210 °C, bevorzugt 45 bis 130 °C, insbesondere bevorzugt 55 bis 95 °C betragen. Da die Beladung des Kühlmittels 4 mit Nebenkomponenten im Laufe der Zeit zunimmt, kann ein Teil des beladenen Kühlmittels aus dem Umlauf abgezogen werden (4a) und die Umlaufmenge durch Zugabe von unbeladenem Kühlmittel (4b) konstant gehalten werden. Das Verhältnis von Ablaufmenge und Zugabemenge hängt von der Dampfbeladung des Produktgases und der Produktgastemperatur am Ende der erste Quenchstufe ab. Je nach Temperatur, Druck und Wassergehalt des Produktgases 2 kann es in der ersten Quenchstufe 3 zur Kondensation von Wasser kommen. In diesem Falle kann sich eine zusätzliche wässrige Phase 5 bilden, welche zusätzlich wasserlösliche Nebenkomponenten enthalten kann. Diese kann dann im Sumpf der Quenchstufe 3 abgezogen werden. Bevorzugt ist ein Betrieb, in dem sich in der ersten Quenchstufe 3 keine wässrige Phase ausbildet.

Der abgekühlte und eventuell an Nebenkomponenten abgereicherte Produktgasstrom 6 kann nun einer zweiten Quenchstufe 7 zugeführt werden. In dieser kann er nun erneut mit einem Kühlmittel 8 in Kontakt gebracht werden.

Als Kühlmittel 8 können Wasser, alkalisch-wässrige Lösungen, organische Lösungsmittel oder eine Kombination oder Gemische daraus verwendet werden. Vorzugsweise wird ein organisches Lösungsmittel verwendet. Als ein oben genanntes Lösungsmittel kann, solange es in der Lage ist, Teile der im Gasstrom befindlichen Nebenkomponenten aufzunehmen, jedes Lösungsmittel verwendet werden. Bevorzugt sind aromatische Kohlenwasserstoff- Lösungsmittel wie Toluol oder Mesitylen, da in diesen die Löslichkeitsgrenze größer 1000 ppm mg aktiver Sauerstoff / kg Lösungsmittel ist.

Im Allgemeinen wird das Produktgas bis zum Gasausgang der zweiten Quenchstufe 7 auf 5 bis 100 °C, vorzugsweise auf 15 bis 85 °C und noch mehr bevorzugt auf 30 bis 70 °C gekühlt. Das Kühlmittel kann im Gegenstrom zum Produktgas zugeführt werden. In diesem Fall kann die Temperatur des Kühlmittelmediums 8 am Kühlmitteleinlass 5 bis 100 °C, bevorzugt 15 bis 85 °C, insbesondere bevorzugt 30 bis 70 °C betragen. Der Druck in der zweiten Quenchstufe 7 ist nicht besonders eingeschränkt, beträgt aber im Allgemeinen 0,01 bis 4 bar (ü), bevorzugt 0,1 bis 2 bar (ü) und besonders bevorzugt 0,2 bis 1 bar (ü). Das im Kühlturm der zweiten Quenchstufe eingesetzte Kühlmittel 8 wird zirkulierend eingesetzt. Der Kreislaufstrom des Kühlmittels 8 in Liter pro Stunde bezogen auf den Massenstrom an Butadien in Gramm pro Stunde kann im Allgemeinen 0,0001 bis 5 l/g, bevorzugt 0,001 bis 1 l/g und besonders bevorzugt 0,002 bis 0,2 l/g betragen.

Je nach Temperatur, Druck und Wassergehalt des Produktgases 6 kann es in der zweiten Quenchstufe 7 zur Kondensation von Wasser kommen. In diesem Falle kann sich eine zusätzliche wässrige Phase 9 bilden, welche zusätzlich wasserlösliche Nebenkomponenten enthalten kann. Diese kann dann im Sumpf der Quenchstufe 7 abgezogen werden. Befindet sich im Sumpf der zweiten Qenchstufe 7 eine wässrige Phase 9 oder wenn in einem Teil des Quenches Wasser als Kühlmittel eingesetzt wird, gelingt das Einlösen von Nebenprodukten der ODH-Reaktion, zum Beispiel Essigsäure, MSA, etc. bei erhöhtem pH-Wert besser als bei niedrigem pH-Wert. Da das Einlösen von Nebenprodukten wie den oben genannten den pH-Wert von zum Beispiel Wasser erniedrigt, kann der pH-Wert durch Zugabe eines alkalischen Mediums konstant gehalten oder erhöht werden. Im Allgemeinen wird der pH-Wert der wässrigen Phase im Sumpf der zweiten Quenchstufe 7 zwischen 1 bis 14, bevorzugt zwischen 2 bis 12, besonders bevorzugt zwischen 3 bis 11 gehalten. Je saurer der Wert, desto weniger alkalisches Medium muss zugeführt werden. Je basischer, desto besser gelingt die Einlösung einiger Nebenprodukte. Jedoch führen sehr hohe pH-Werte zum Einlösen von Nebenprodukten wie CO₂ und damit zu einem sehr hohen Verbrauch des alkalischen Mediums. Die Temperatur des Kühlmittels 8 im Sumpf kann im Allgemeinen 20 bis 210 °C, bevorzugt 35 bis 120 °C, insbesondere bevorzugt 45 bis 85 °C betragen. Da die Beladung des Kühlmittels 8 mit Nebenkomponenten im Laufe der Zeit zunimmt, kann ein Teil des beladenen Kühlmittels (8a) aus dem Umlauf abgezogen werden und die Umlaufmenge durch Zugabe von unbeladenem Kühlmittel (8b) konstant gehalten werden.

Um einen möglichst guten Kontakt von Produktgas und Kühlmittel zu erreichen, können Einbauten in der zweiten Quenchstufe vorhanden sein. Solche Einbauten umfassen zum Beispiel Glocken-, Zentrifugal- und/oder Siebböden, Kolonnen mit strukturierten Packungen, z.B. Blechpackungen mit einer spezifischen Oberfläche von 100 bis 1000 m²/m³ wie Mellapak® 250 Y, und Füllkörperkolonnen.

Die Umläufe der beiden Quenchstufen können sowohl voneinander getrennt als auch miteinander verbunden sein. So kann beispielsweise der Strom 8a dem Strom 4b zugeführt werden oder diesen ersetzen. Die gewünschte Temperatur der Umlaufströme kann über geeignete Wärmetauscher eingestellt werden.

Um den Mitriss von flüssigen Bestandteilen aus dem Quench in die Abgasleitung zu minimieren, können geeignete bauliche Maßnahmen, wie zum Beispiel der Einbau eines Demisters, getroffen werden. Weiterhin können hochsiedende Substanzen, welche im Quench nicht vom Produktgas abgetrennt werden, durch weitere bauliche Maßnahmen, wie beispielsweise weitere Gaswäschen, aus dem Produktgas entfernt werden. Es wird ein Gasstrom 10 erhalten, in welchem n-Butan, 1-Buten, 2-Butene, Butadien, gegebenenfalls Sauerstoff, Wasserstoff, Wasserdampf, in geringen Mengen Methan, Ethan, Ethen, Propan und Propen, iso-Butan, Kohlenstoffoxide, Inertgase und Teile des im Quench verwendeten Lösungsmittel verbleiben. Weiterhin können in diesem Produktgasstrom Spuren von hochsiedenden Komponenten verbleiben, welche im Quench nicht quantitativ abgetrennt wurden.

Anschließend wird der Produktgasstrom 10 aus dem Quench in mindestens einer Kompressionsstufe 11 komprimiert und nachfolgend in der Kühlstufe 13 weiter abgekühlt, wobei mindestens ein Kondensatstrom enthaltend Wasser 15 und das im Quench verwendete Lösungsmittel 14 auskondensieren, und ein Gasstrom16 enthaltend Butadien, 1-Buten, 2-Butene, Sauerstoff, Wasserdampf, gegebenenfalls leichtsiedende Kohlenwasserstoffe wie Methan, Ethan, Ethen, Propan und Propen, Butan und iso-Butan, gegebenenfalls Kohlenstoffoxide und gegebenenfalls Inertgase verbleiben. Weiterhin können in diesem Produktgasstrom Spuren von hochsiedenden Komponenten verbleiben.

Die Kompression und Kühlung des Gasstroms 10 kann ein- oder mehrstufig (n-stufig) erfolgen. Im Allgemeinen wird insgesamt von einem Druck im Bereich von 1,0 bis 4,0 bar (absolut) auf einen Druck im Bereich von 3,5 bis 20 bar (absolut) komprimiert. Nach jeder Kompressionsstufe folgt eine Abkühlstufe, in der der Gasstrom auf eine Temperatur im Bereich von 15 bis 60 °C abgekühlt wird. Der Kondensatstrom kann somit bei mehrstufiger Kompression auch mehrere Ströme umfassen. Der Kondensatstrom besteht zu großen Teilen aus Wasser 15 und dem im Quench verwendeten Lösungsmittel 16. Beide Ströme können daneben in geringem Umfang Leichtsieder, C₄-Kohlenwasserstoffe, Oxygenate und Kohlenstoffoxide enthalten.

Die Abkühlstufen sind vorzugsweise als Direktkühler ausgebildet. Diese Direktkühler werden als vertikale Kolonnen mit Einbauten ausgeführt. Als Einbauten kommen Schüttungen wie z.B. Pallringe, Packungen oder vorzugsweise Kolonnenböden wie z.B. Siebböden, Kaskadenböden oder Ventilböden in Frage. Ein flüssiges Kühlmedium wie zum Beispiel Wasser oder ein Kohlenwasserstoff wie Toluol, bevorzugt Mesitylen wird im Gleichstrom oder bevorzugt im Gegenstrom zum zu kühlenden Gasstrom geführt.

Um den Strom 12 zu kühlen und/oder um weitere Nebenkomponenten aus dem Strom 12 zu entfernen, kann das kondensierte, im Quench verwendete Lösungsmittel 14 in einem Wärmetauscher abgekühlt und in die Kühlstufe 13 rückgeführt werden. Da die Beladung des Kühlmittels 14 mit Nebenkomponenten im Laufe der Zeit zunimmt, kann ein Teil des beladenen Kühlmittels 14a aus dem Umlauf abgezogen werden und die Umlaufmenge durch Zugabe von unbeladenem Kühlmittel 14b konstant gehalten werden.

Der Kondensatstrom 14a kann in den Kreislaufstrom 4b und/oder 8b des Quenches zurückgeführt werden. Dadurch können die im Kondensatstrom 14a absorbierten C₄-Komponenten wieder in den Gasstrom gebracht und damit die Ausbeute erhöht werden.

Geeignete Verdichter sind beispielsweise Turbo-, Drehkolben- und Hubkolbenverdichter. Die Verdichter können beispielsweise mit einem Elektromotor, einem Expander oder einer Gas- oder Dampfturbine angetrieben werden. Typische Verdichtungsverhältnisse (Austrittsdruck : Eintrittsdruck) pro Verdichterstufe liegen je nach Bauart zwischen 1,5 und 3,0. Die Abkühlung des verdichteten Gases erfolgt mit Wärmetauschern, die beispielsweise als Rohrbündel-, Spiral- oder Plattenwärmetauscher oder als Direktkühler ausgeführt sein können.

Die Gehäuse der Verdichter sind vorzugsweise explosionsfest ausgelegt. Hierbei werden die Gehäuse der Verdichter entsprechend den zu erwartenden Explosionsdrücken ausgelegt. Beispielsweise beträgt der maximale Betriebsdruck eines Verdichters 5 bar, der Explosionsdruckfaktor des im Verdichter befindlichen Gasgemisches beträgt 10. Damit ergibt sich für die mechanische Auslegung des Verdichtergehäuses ein Designdruck von 50 bar. Werkstoff sowie Wandstärke werden dann gemäß dem Designdruck von 50 bar gewählt.

Der Butadien, n-Butene, Sauerstoff, leicht siedende Kohlenwasserstoffe (Methan, Ethan, Ethen, Propan, Propen, n-Butan, iso-Butan), gegebenenfalls Wasserdampf, gegebenenfalls Kohlenstoffoxide sowie gegebenenfalls Inertgase enthaltende Gasstrom 16 wird als Ausgangsstrom der weiteren Aufbereitung zugeführt.

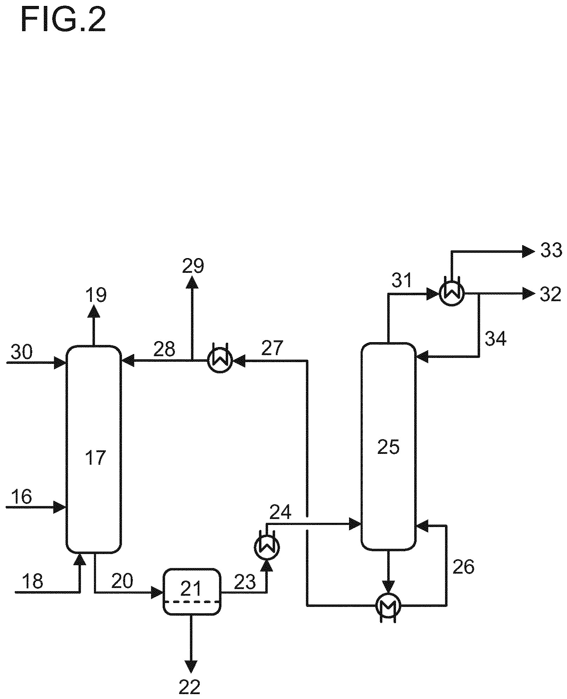

In einem Schritt D) (Figur 2) werden nicht kondensierbare und leicht siedende Gasbestandteile, umfassend Sauerstoff, leicht siedenden Kohlenwasserstoffe (Methan, Ethan, Ethen, Propan, Propen), Kohlenstoffoxide und Inertgase in einer Absorptionskolonne 17 als Gasstrom 19 aus dem Prozessgasstrom 16 durch Absorption der C₄-Kohlenwasserstoffe in einem hochsiedenden Absorptionsmittel (28 und/oder 30) und nachfolgender Desorption der C₄-Kohlenwasserstoffe abgetrennt. Dieser Schritt D) umfasst bevorzugt die Teilschritte
Da) Absorption der C₄-Kohlenwasserstoffe umfassend Butadien und n-Butene in einem hochsiedenden Absorptionsmittel (28 und/oder 30), wobei ein mit C₄-Kohlenwasserstoffen beladener Absorptionsmittelstrom und der Gasstrom 19 erhalten werden,
Db) Entfernung von Sauerstoff aus dem mit C₄-Kohlenwasserstoffen beladenen Absorptionsmittelstrom aus Schritt Da) durch Strippung mit einem nicht kondensierbaren Gasstrom 18, wobei ein mit C₄-Kohlenwasserstoffen beladener Absorptionsmittelstrom 20 erhalten wird und
Dc) Desorption der C₄-Kohlenwasserstoffe aus dem beladenen Absorptionsmittelstrom, wobei ein C₄-Produktgasstrom 31 erhalten wird.

Dazu wird in der Absorptionsstufe 17 der Gasstrom 16 mit einem inerten Absorptionsmittel in Kontakt gebracht und werden die C₄-Kohlenwasserstoffe in dem inerten Absorptionsmittel absorbiert, wobei ein mit C₄-Kohlenwasserstoffen beladenes Absorptionsmittel 20 und ein die übrigen Gasbestandteile enthaltendes Abgas 19 erhalten werden. In einer Desorptionsstufe werden die C₄-Kohlenwasserstoffe aus dem hochsiedenden Absorptionsmittel wieder freigesetzt.

Die Absorptionsstufe kann in jeder beliebigen, dem Fachmann bekannten geeigneten Absorptionskolonne durchgeführt werden. Die Absorption kann durch einfaches Durchleiten des Produktgasstroms durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Bevorzugt wird die Absorption im Gegenstrom durchgeführt. Geeignete Absorptionskolonnen sind z.B. Bodenkolonnen mit Glocken-, Zentrifugal- und/oder Siebboden, Kolonnen mit strukturierten Packungen, z.B. Blechpackungen mit einer spezifischen Oberfläche von 100 bis 1000 m²/m³ wie Mellapak® 250 Y, und Füllkörperkolonnen. Es kommen aber auch Riesel- und Sprühtürme, Graphitblockabsorber, Oberflächenabsorber wie Dickschicht und Dünnschichtabsorber sowie Rotationskolonnen, Tellerwäscher, Kreuzschleierwäscher und Rotationswäscher in Betracht.

Vorzugsweise ist die Absorptionskolonne eine Bodenkolonne, die explosionsfest ausgelegt ist. Hierbei wird der Mantel der Kolonne entsprechend dem zu erwartenden Explosionsdruck ausgelegt. Beispielsweise beträgt der maximale Betriebsdruck der Kolonne 10 bar, der Explosionsdruckfaktor des in der Kolonne befindlichen Gasgemisches beträgt 9. Damit ergibt sich für die mechanische Auslegung des Kolonnenmantels ein Designdruck von 90 bar. Werkstoff sowie Wandstärke werden dann gemäß dem Designdruck von 90 bar gewählt.

Vorzugsweise weist die Absorptionskolonne gasdichte Kolonnenböden auf. Die gasführenden Zuleitungen in die Absorptionskolonne tauchen vorzugsweise in die Flüssigkeit ein. Damit wird ein Fortschreiten der Flammenfront in die Kolonne hinein verhindert.

In einer Ausführungsform wird einer Absorptionskolonne im unteren Bereich der Butadien, n-Butene und die leichtsiedenden und nicht kondensierbaren Gasbestandteile enthaltende Gasstrom16 zugeführt. Im oberen Bereich der Absorptionskolonne wird das hochsiedende Absorptionsmittel (28 und/oder 30) aufgegeben.

In der Absorptionsstufe eingesetzte inerte Absorptionsmittel sind im Allgemeinen hochsiedende unpolare Lösungsmittel, in denen das abzutrennende C₄-Kohlenwasserstoff-Gemisch eine deutlich höhere Löslichkeit als die übrigen abzutrennenden Gasbestandteile aufweist. Geeignete Absorptionsmittel sind vergleichsweise unpolare organische Lösungsmittel, beispielsweise aliphatische C₈- bis C₁₈-Alkane, oder aromatische Kohlenwasserstoffe wie die Mittelölfraktionen aus der Paraffindestillation, Toluol oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁-C₈-Alkanolen, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl®. Häufig enthält dieses Lösungsmittelgemisch Dimethylphthalat in einer Menge von 0,1 bis 25 Gew.-%.

Bevorzugte Absorptionsmittel sind Lösungsmittel, die ein Lösungsvermögen für organische Peroxide von mindestens 1000 ppm (mg aktiver Sauerstoff / kg Lösungsmittel) aufweisen. In der bevorzugten Ausführungsform wird als Lösungsmittel für die Absorption Toluol, Xylol oder Mesitylen eingesetzt.

Am Kopf der Absorptionskolonne 17 wird ein Abgasstrom 19 abgezogen, der im Wesentlichen Sauerstoff, leicht siedende Kohlenwasserstoffe (Methan, Ethan, Ethen, Propan, Propen), gegebenenfalls C₄-Kohlenwasserstoffe (Butan, Butene, Butadien), gegebenenfalls Inertgase, gegebenenfalls Kohlenstoffoxide und gegebenenfalls noch Wasserdampf enthält. Dieser Stoffstrom kann teilweise dem ODH-Reaktor zugeführt werden. Damit lässt sich zum Beispiel der Eintrittsstrom des ODH-Reaktors auf den gewünschten C₄-Kohlenwasserstoffgehalt einstellen.

Am Sumpf der Absorptionskolonne werden in einer weiteren Kolonne durch die Spülung mit einem Gas 18 Reste von im Absorptionsmittel gelöstem Sauerstoff ausgetragen. Der verbleibende Sauerstoffanteil ist vorzugsweise so gering, dass der die Desorptionskolonne verlassende, Butan, Buten sowie Butadien enthaltende Strom 31 nur noch maximal 100 ppm Sauerstoff enthält.

Das Ausstrippen des Sauerstoffs kann in jeder beliebigen, dem Fachmann bekannten geeigneten Kolonne durchgeführt werden. Das Strippen kann durch einfaches Durchleiten von nicht kondensierbaren Gasen durch die beladene Absorptionslösung erfolgen. Mit ausgestrippte C4-Kohlenwasserstoffe werden im oberen Teil der Absorptionskolonne 17 zurück in die Absorptionslösung gewaschen, indem der Gasstrom in diese Absorptionskolonne zurück geleitet wird. Das kann sowohl durch eine Verrohrung der Stripperkolonne als auch eine direkte Montage der Stripperkolonne unterhalb der Absorberkolonne erfolgen. Da der Druck im Strippkolonnenteil und Absorptionskolonnenteil erfindungsgemäß gleich ist, kann diese direkte Kopplung erfolgen. Geeignete Stippkolonnen sind z.B. Bodenkolonnen mit Glocken-, Zentrifugal- und/oder Siebboden, Kolonnen mit strukturierten Packungen, z.B. Blechpackungen mit einer spezifischen Oberfläche von 100 bis 1000 m²/m³ wie Mellapak® 250 Y, und Füllkörperkolonnen. Es kommen aber auch Riesel- und Sprühtürme sowie Rotationskolonnen, Tellerwäscher, Kreuzschleierwäscher und Rotationswäscher in Betracht. Geeignete Gase sind zum Beispiel Stickstoff oder Methan.

Der mit C₄-Kohlenwasserstoffen beladene Absorptionsmittelstrom 20 beinhaltet Wasser. Dieses wird in einem Dekanter 21 als Strom 22 vom Absorptionsmittel abgetrennt, so dass ein Strom 23 erhalten wird, der nur noch das im Absorptionsmittel eingelöste Wasser enthält.

Der mit C₄-Kohlenwasserstoffen beladene, weitestgehend vom Wasser befreite Absorptionsmittelstrom 23 kann in einem Wärmetauscher erwärmt werden und als Strom 24 anschließend in eine Desorptionskolonne 25 geleitet werden. In einer Verfahrensvariante wird der Desorptionsschritt Dc) durch Entspannung und/oder Erhitzen des beladenen Absorptionsmittels durchgeführt. Bevorzugte Verfahrensvariante ist die Nutzung eines Reboilers im Sumpf der Desorptionskolonne 25.

Vorzugsweise ist die Desorptionskolonne eine Bodenkolonne, deren Mantel wie oben bereits beschrieben explosionsfest ausgelegt ist. Vorzugsweise weist die Desorptionskolonne gasdichte Kolonnenböden auf. Die gasführenden Zuleitungen in die Desorptionskolonne tauchen vorzugsweise in die Flüssigkeit ein. Damit wird ein Fortschreiten der Flammenfront in die Kolonne hinein verhindert.

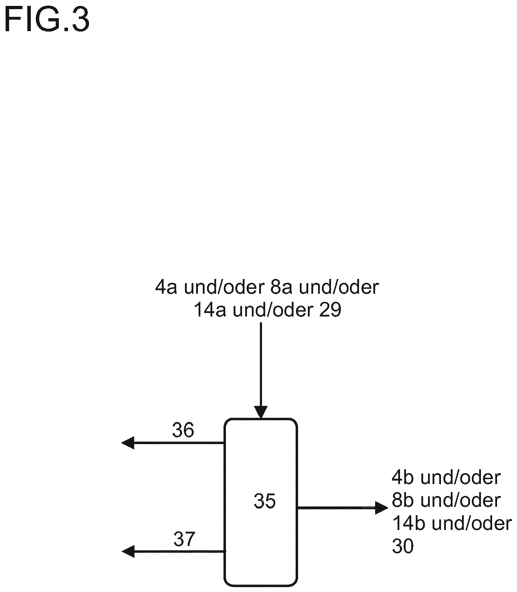

Das in der Desorptionsstufe regenerierte Absorptionsmittel 27 kann in einem Wärmetauscher abgekühlt werden und als Strom 28 in die Absorptionsstufe 17 zurückgeführt werden. Im Prozessgasstrom befindliche Leichtsieder wie beispielsweise Ethan oder Propan sowie schwersiedende Komponenten wie Benzaldehyd, Maleinsäure und Phthalsäure, können sich im Kreislaufstrom anreichern. Um die Anreicherung zu begrenzen, kann ein Purgestrom 29 abgezogen werden und dieser entweder wie auch die Ströme 14a, 8b und 4b in einer Destillationskolonne 35 (Figur 3) nach dem Stand der Technik in Leichtsieder 36, regeneriertes Absorbens 30 (Figur 2 und 3) und Schwersieder 37 aufgetrennt werden, oder bevorzugt den Strömen 14b, 8b oder 4b zugeführt werden, um die noch im Strom 29 gelösten C₄-Kohlenwasserstoffe in den Prozessgasstrom zurückzuwaschen. Wenn Strom 29 in der Destillationskolonne 35 getrennt wird, können die Ströme 36 und 37verbrannt und somit energetisch genutzt werden.

Der im Wesentlichen aus n-Butan, n-Butenen und Butadien bestehende C₄-Produktgasstrom 31 enthält im Allgemeinen 20 bis 80 Vol.-% Butadien, 0 bis 80 Vol.-% n-Butan, 0 bis 10 Vol.-% 1-Buten, und 0 bis 50 Vol.-% 2-Butene, wobei die Gesamtmenge 100 Vol.-% ergibt. Weiterhin können geringe Mengen an iso-Butan enthalten sein.

Ein Teil des kondensierten, hauptsächlich C₄-Kohlenwasserstoffe enthaltenden Kopfaustrags der Desorptionskolonne wird als Strom 34 in den Kolonnenkopf zurückgeführt, um die Trennleistung der Kolonne zu erhöhen.

Die C₄-Produktströme 32 und 33 werden anschließend durch Extraktivdestillation im Schritt E) mit einem für Butadien selektiven Lösungsmittel in einen Butadien und das selektive Lösungsmittel enthaltenden Stoffstrom und einen n-Butene enthaltenden Stoffstrom aufgetrennt.

Die Extraktivdestillation kann beispielsweise, wie in "Erdöl und Kohle - Erdgas - Petrochemie", Band 34 (8), Seiten 343 bis 346 oder "Ullmanns Enzyklopädie der Technischen Chemie", Band 9, 4. Auflage 1975, Seiten 1 bis 18 beschrieben, durchgeführt werden. Hierzu wird der C₄-Produktgasstrom mit einem Extraktionsmittel, vorzugsweise einem N-Methylpyrrolidon (NMP)/Wasser-Gemisch, in einer Extraktionszone in Kontakt gebracht. Die Extraktionszone ist im Allgemeinen in Form einer Waschkolonne ausgeführt, welche Böden, Füllkörper oder Packungen als Einbauten enthält. Diese weist im Allgemeinen 30 bis 70 theoretische Trennstufen auf, damit eine hinreichend gute Trennwirkung erzielt wird. Vorzugsweise weist die Waschkolonne im Kolonnenkopf eine Rückwaschzone auf. Diese Rückwaschzone dient zur Rückgewinnung des in der Gasphase enthaltenen Extraktionsmittels mit Hilfe eines flüssigen Kohlenwasserstoffrücklaufs, wozu die Kopffraktion zuvor kondensiert wird. Das Massenverhältnis Extraktionsmittel zu C₄-Produktgasstrom im Zulauf der Extraktionszone beträgt im Allgemeinen 10 : 1 bis 20 : 1. Die Extraktivdestillation wird vorzugsweise bei einer Sumpftemperatur im Bereich von 100 bis 250 °C, insbesondere bei einer Temperatur im Bereich von 110 bis 210 °C, einer Kopftemperatur im Bereich von 10 bis 100·°C, insbesondere im Bereich von 20 bis 70·°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

Die Extraktivdestillationskolonne ist vorzugsweise wie oben beschrieben explosionsfest ausgelegt. Die Extraktivdestillationskolonne ist vorzugsweise eine Bodenkolonne mit gasdichten Kolonnenböden. Die gasführenden Zuleitungen in die Extraktivdestillationskolonne tauchen vorzugsweise in die Flüssigkeit ein.

Geeignete Extraktionsmittel sind Butyrolacton, Nitrile wie Acetonitril, Propionitril, Methoxypropionitril, Ketone wie Aceton, Furfural, N-alkylsubstituierte niedere aliphatische Säureamide wie Dimethylformamid, Diethylformamid, Dimethylacetamid, Diethylacetamid, N-Formylmorpholin, N-alkylsubstituierte zyklische Säureamide (Lactame) wie N-Alkylpyrrolidone, insbesondere N-Methylpyrrolidon (NMP). Im Allgemeinen werden alkylsubstituierte niedere aliphatische Säureamide oder N-alkylsubstituierte zyklische Säureamide verwendet. Besonders vorteilhaft sind Dimethylformamid, Acetonitril, Furfural und insbesondere NMP.

Es können jedoch auch Mischungen dieser Extraktionsmittel untereinander, z.B. von NMP und Acetonitril, Mischungen dieser Extraktionsmittel mit Co-Lösungsmitteln und/oder tert.-Butylether, z.B. Methyl-tert.-butylether, Ethyl-tert.-butylether, Propyl-tert.-butylether, n- oder iso-Butyl-tert.-butylether eingesetzt werden. Besonders geeignet ist NMP, bevorzugt in wässriger Lösung, vorzugsweise mit 0 bis 20 Gew.-% Wasser, besonders bevorzugt mit 7 bis 10 Gew.-% Wasser, insbesondere mit 8,3 Gew.-% Wasser.

Der Kopfproduktstrom der Extraktivdestillationskolonne enthält im Wesentlichen Butan und Butene und in geringen Mengen Butadien und wird gasförmig oder flüssig abgezogen. Im Allgemeinen enthält der im Wesentlichen aus n-Butan und 2-Buten bestehende Strom bis 100 Vol.-% n-Butan, 0 bis 50 Vol.-% 2-Buten und 0 bis 3 Vol.-% weitere Bestandteile wie Isobutan, Isobuten, Propan, Propen und C₅⁺-Kohlenwasserstoffe.

Der im Wesentlichen aus n-Butan und 2-Buten bestehende Strom kann ganz oder teilweise dem C₄-Feed des ODH-Reaktors zugeführt werden. Da die Buten-Isomere dieses Rückführstroms im Wesentlichen aus 2-Butenen bestehen, und 2-Butene im Allgemeinen langsamer zu Butadien oxidativ dehydriert werden als 1-Buten, kann dieser Rückführstrom vor der Zuführung in den ODH-Reaktor katalytisch isomerisiert werden. Dadurch kann die Isomerenverteilung entsprechend der im thermodynamischen Gleichgewicht vorliegenden Isomerenverteilung eingestellt werden.

In einem Schritt F) wird der Butadien und das selektive Lösungsmittel enthaltende Stoffstrom in einen im Wesentlichen aus dem selektiven Lösungsmittel bestehenden Stoffstrom und einen Butadien enthaltenden Stoffstrom destillativ aufgetrennt.

Der am Sumpf der Extraktivdestillationskolonne gewonnene Stoffstrom enthält im Allgemeinen das Extraktionsmittel, Wasser, Butadien und in geringen Anteilen Butene und Butan und wird einer Destillationskolonne zugeführt. In dieser kann über Kopf Butadien gewonnen werden. Am Sumpf der Destillationskolonne fällt ein Extraktionsmittel und gegebenenfalls Wasser enthaltender Stoffstrom an, wobei die Zusammensetzung des Extraktionsmittel und Wasser enthaltenden Stoffstroms der Zusammensetzung entspricht, wie sie der Extraktion zugegeben wird. Der Extraktionsmittel und Wasser enthaltende Stoffstrom wird bevorzugt in die Extraktivdestillation zurückgeleitet.

In einer Variante wird über einen Seitenabzug ein Butadien enthaltendes Extraktionsmittel abgezogen und in eine Desorptionszone überführt, wobei aus der Extraktionsmittellösung das Butadien desorbiert wird. Die Desorptionszone kann beispielsweise in Form einer Waschkolonne ausgeführt sein, die 2 bis 30, bevorzugt 5 bis 20 theoretische Stufen und gegebenenfalls eine Rückwaschzone mit beispielsweise 4 theoretischen Stufen aufweist. Diese Rückwaschzone dient zur Rückgewinnung des in der Gasphase enthaltenen Extraktionsmittels mit Hilfe eines flüssigen Kohlenwasserstoffrücklaufs aus Butadien, wozu die Kopffraktion kondensiert wird. Als Einbauten sind Packungen, Böden oder Füllkörper vorgesehen. Die Destillation wird vorzugsweise bei einer Sumpftemperatur im Bereich von 100 bis 300 °C, insbesondere im Bereich von 150 bis 200 °C und einer Kopftemperatur im Bereich von 0 bis 70 °C, insbesondere im Bereich von 10 bis 50 °C durchgeführt. Der Druck in der Destillationskolonne liegt dabei vorzugsweise im Bereich von 1 bis 10 bar. Im Allgemeinen herrschen in der Desorptionszone gegenüber der Extraktionszone ein verminderter Druck und/oder eine erhöhte Temperatur.

Der am Kolonnenkopf gewonnene Wertproduktstrom enthält im Allgemeinen 90 bis 100 Vol.-% Butadien, 0 bis 10 Vol.-% 2-Buten und 0 bis 10 Vol.-% n-Butan und iso-Butan. Zur weiteren Aufreinigung des Butadiens kann eine weitere Destillation nach dem Stand der Technik durchgeführt werden.

Der Mantel der Destillationskolonne ist vorzugsweise wie oben bereits beschrieben explosionsfest ausgelegt. Die Destillationskolonne ist vorzugsweise eine Bodenkolonne mit gasdichten Kolonnenböden. Die gasführenden Zuleitungen in die Destillationskolonne tauchen vorzugsweise in die Flüssigkeit ein um ein Fortschreiten der Flammenfront in die Kolonne hinein zu verhindern.

Die Erfindung betrifft auch eine Vorrichtung zur Durchführung des Verfahrens zur Herstellung von Butadien aus n-Butenen umfassend:
A) Zuleitung zur Bereitstellung eines n-Butene enthaltenden Einsatzgasstroms a;
B) Zuleitungen zur Einspeisung des n-Butene enthaltenden Einsatzgasstromes a und eines sauerstoffhaltigen Gases in mindestens eine Dehydrierzone, Dehydrierzone umfassend einen Dehydrierreaktor zur oxidativen Dehydrierung von n-Butenen zu Butadien, wobei ein Produktgasstrom b enthaltend Butadien, nicht umgesetzte n-Butene, Wasserdampf, Sauerstoff, leicht siedende Kohlenwasserstoffe, gegebenenfalls Kohlenstoffoxide und gegebenenfalls Inertgase erhalten wird;
C) Zuleitung zur Einspeisung des Produktgasstroms b in eine Abkühlstufe und mindestens eine Abkühlstufe umfassend mindestens eine Quenchkolonne und mindestens eine Kompressionsstufe umfassend mindestens einen Kompressor und gegebenenfalls einen oder mehrere Zwischenkühler zwischen den Kompressoren, wobei der Produktgasstrom b mit mindestens einem im Kreis geführtem Kühlmittel in Kontakt gebracht wird, wobei mindestens ein Kondensatstrom c1 enthaltend Wasser und ein Gasstrom c2 enthaltend Butadien, n-Butene, Wasserdampf, Sauerstoff, leicht siedende Kohlenwasserstoffe, gegebenenfalls Kohlenstoffoxide und gegebenenfalls Inertgase erhalten wird;
D) Einrichtung zur Abtrennung von nicht kondensierbaren und leicht siedenden Gasbestandteilen umfassend Sauerstoff, leicht siedende Kohlenwasserstoffe, gegebenenfalls Kohlenstoffoxide und gegebenenfalls Inertgase als Gasstrom d2 aus dem Gasstrom c2 durch Absorption der C₄-Kohlenwasserstoffe umfassend Butadien und n-Butene in mindestens einem im Kreis geführten Absorptionsmittel, wobei mindestens ein mit C₄-Kohlenwasserstoffen beladener Absorptionsmittelstrom und der Gasstrom d2 erhalten werden, und Einrichtung zur anschließenden Desorption der C₄-Kohlenwasserstoffe aus dem beladenen Absorptionsmittelstrom, wobei ein C₄-Produktgasstrom d1 erhalten wird;
E) Extraktivdestillationskolonne zur Auftrennung des C₄-Produktstroms d1 durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2;
F) Destillationskolonne zur Destillation des Butadien und das selektive Lösungsmittel enthaltenden Stoffstroms e1 in einen im Wesentlichen aus dem selektiven Lösungsmittel bestehenden Stoffstrom f1 und einen Butadien enthaltenden Stoffstrom f2;
dadurch gekennzeichnet, dass
einer oder mehrere der zur Durchführung der Schritte A) bis F) eingesetzten Apparate explosionsfest ausgeführt sind, flüssigkeitsführende Rohrleitungen explosionsfest ausgeführt sind und Gasleitungen detonationsfest ausgeführt sind, insbesondere sind ein oder mehrere der nachstehenden Apparate explosionsfest ausgelegt:
- der in Schritt B) eingesetzte Dehydrierreaktor;
- die in Schritt C) eingesetzte Quenchkolonne;
- die in Schritt C) eingesetzten Kompressoren;
- die in Schritt C) eingesetzten Zwischenkühler;
- die in Schritt F) eingesetzte Destillationskolonne;
- der in der Lösungsmittelregeneriereinheit eingesetzte Dekanter.

Die Erfindung wird durch das nachstehende Beispiel näher erläutert.

### Beispiel

Eine kommerzielle Anlage zur Erzeugung von 1,3-Butadien aus n-Butenen umfasst unter anderem
- Reaktoren zur katalytischen oxidativen Dehydrierung
- eine Quenchkolonne zur Abkühlung des Produktgasstroms
- Verdichter zur Kompression des Produktgasstroms
- Eine Absorptionskolonne zur Abtrennung der inerten Gasbestandteile als Kopfprodukt von der C4-Fraktion als Sumpfprodukt

Um eine ausreichende Katalysatorstandzeit zu gewährleisten, werden die Reaktoren mit einem Sauerstoffüberschuss von 6 Vol.-% am Reaktoraustritt betrieben. Die Mengenströme am Eintritt in die Reaktoren (n-Butene, Luft, Dampf, Stickstoff) werden mit zertifizierten Mengenmessungen und Verhältnisregelungen ausgestattet. Durch Kondensation von im Produktgas enthaltenem Wasserdampf in der Quenchkolonne erhöht sich die Sauerstoffkonzentration im Kopfprodukt der Absorptionskolonne auf über 7 Vol.-%. Betroffene Apparate in den genannten Anlagensektionen werden explosionsdruckfest ausgelegt, die verbindenden Rohrleitungen detonationsfest ausgelegt. Als zusätzliches Sicherheitselement wird im Kopfprodukt der Absorptionskolonne eine zertifizierte Sauerstoffmessung installiert, mittels der die Sauerstoffkonzentration im System kontinuierlich überwacht werden kann. Als Messprinzipien für diese Analysengeräte kommen Lasermessungen oder paramagnetische Messungen oder eine Kombination beider Messprinzipien in Frage.

Das erfindungsgemäße Sicherheitskonzept ermöglicht die Auslegung und den sicheren Betrieb von kommerziellen Anlagen zur oxidativen Deydrierung von Butenen zu Butadien, insbesondere bei Verfahren, bei denen am Austritt des Oxidationsreaktors ein Sauerstoffüberschuss erforderlich ist.

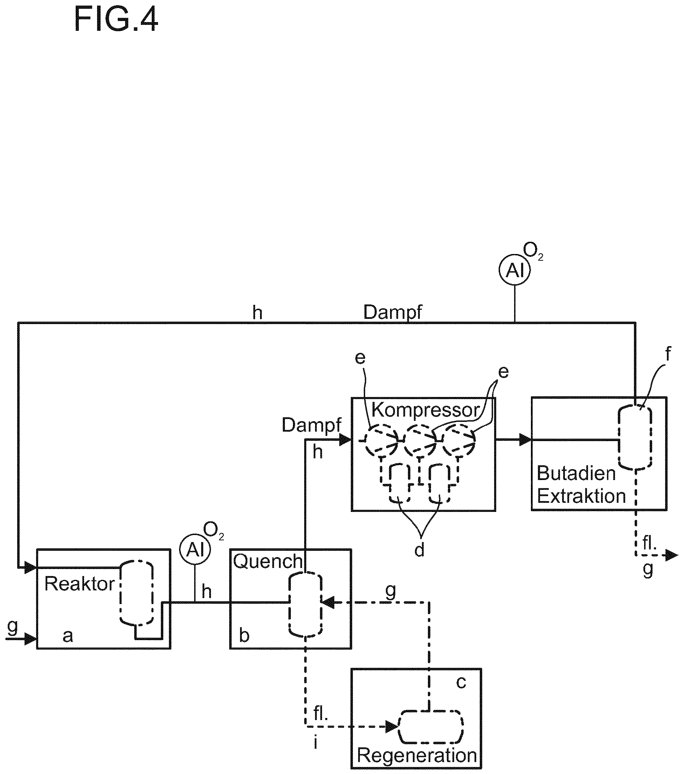

Figur 4 zeigt
a) Reaktor in der Reaktorsektion in explosionsfester Auslegung
b) Quenchkolonne in der Quenchsektion in explosionsfester Auslegung
c) Lösungsmittel-Regenerationseinheit in explosionsfester Auslegung
d) Zwischenkühler mit Mesitylen als Kühlmittel in der Verdichtungssektion als Direktkühler in explosionsfester Auslegung
e) Verdichtungsstufen (Kompressoren) in der Verdichtungssektion in explosionsfester Auslegung
f) Butadienkolonne in der Butadiene-Abtrennungssektion in explosionsfester Auslegung
g) explosionsfeste Gasleitungen
h) detonationsfeste Gasleitungen
i) explosionsfeste Flüssigkeitsleitungen

Bei allen Kolonnen sind die Gaszuführungen in die Flüssigkeit abgetaucht.

Alle Flüssigkeitsrohrleitungen zu und von den Kolonnen sind explosionsfest ausgelegt, um bei einer Explosion in einem Behälter eine mechanische Überlastung der Flüssigkeitsrohrleitungen zu verhindern.

Alle Gasrohrleitungen zwischen den Sektionen sind detonationsfest ausgelegt.

## Patentansprüche

1. Verfahren zur Herstellung von Butadien aus n-Butenen mit den folgenden Schritten:
A) Bereitstellung eines n-Butene enthaltenden Einsatzgasstroms a;
B) Einspeisung des n-Butene enthaltenden Einsatzgasstromes a und eines sauerstoffhaltigen Gases in mindestens eine Dehydrierzone umfassend einen Dehydrierreaktor und oxidative Dehydrierung von n-Butenen zu Butadien, wobei ein Produktgasstrom b enthaltend Butadien, nicht umgesetzte n-Butene, Wasserdampf, Sauerstoff, leicht siedende Kohlenwasserstoffe, gegebenenfalls Kohlenstoffoxide und gegebenenfalls Inertgase erhalten wird;
C) Abkühlung und Kompression des Produktgasstroms b in mindestens einer Abkühlstufe umfassend mindestens eine Quenchkolonne und in mindestens einer Kompressionsstufe umfassend mindestens einen Kompressor und gegebenenfalls einen oder mehrere Zwischenkühler zwischen den Kompressoren, wobei der Produktgasstrom b mit mindestens einem im Kreis geführtem Kühlmittel in Kontakt gebracht wird, wobei mindestens ein Kondensatstrom c1 enthaltend Wasser und ein Gasstrom c2 enthaltend Butadien, n-Butene, Wasserdampf, Sauerstoff, leicht siedende Kohlenwasserstoffe, gegebenenfalls Kohlenstoffoxide und gegebenenfalls Inertgase erhalten wird;
D) Abtrennung von nicht kondensierbaren und leicht siedenden Gasbestandteilen umfassend Sauerstoff, leicht siedende Kohlenwasserstoffe, gegebenenfalls Kohlenstoffoxide und gegebenenfalls Inertgase als Gasstrom d2 aus dem Gasstrom c2 durch Absorption der C₄-Kohlenwasserstoffe umfassend Butadien und n-Butene in mindestens einem im Kreis geführten Absorptionsmittel, wobei mindestens ein mit C₄-Kohlenwasserstoffen beladener Absorptionsmittelstrom und der Gasstrom d2 erhalten werden, und anschließende Desorption der C₄-Kohlenwasserstoffe aus dem beladenen Absorptionsmittelstrom, wobei ein C₄-Produktgasstrom d1 erhalten wird;
E) Auftrennung des C₄-Produktstroms d1 durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2;
F) Destillation des Butadien und das selektive Lösungsmittel enthaltenden Stoffstroms e1 in einen im Wesentlichen aus dem selektiven Lösungsmittel bestehenden Stoffstrom f1 und einen Butadien enthaltenden Stoffstrom f2;
**dadurch gekennzeichnet, dass** die nachstehenden Maßnahmen (i) bis (iii) durchgeführt werden:
(i) Vermeidung der Bildung explosionsfähiger Gasgemische durch Überwachung der Sauerstoffkonzentration in den in die Dehydrierzone eingespeisten sauerstoffhaltigen Gasströme und Steuerung der Massenströme von sauerstoffhaltigen Gasströmen und Kohlenwasserstoffe enthaltenden Gasströmen derart, dass sich keine explosionsfähigen Gasgemische ausbilden können;
(ii) Unterbrechung der Zufuhr des sauerstoffhaltigen Gasgemischs in die Dehydrierzone bei Überschreitung eines Grenzwertes für die Sauerstoffkonzentration in dem Deyhdriergasgemisch;
(iii) Durchführung der Schritte A) bis F) in Apparaten, die explosionsfest ausgeführt sind, wobei flüssigkeitsführende Rohrleitungen explosionsfest und Gasleitungen detonationsfest ausgeführt sind.

2. Verfahren nach Anspruch 1, **dadurch gekennzeichnet, dass** einer oder mehrere der nachstehenden Apparate explosionsfest ausgelegt sind:
- der in Schritt B) eingesetzte Dehydrierreaktor;
- die in Schritt C) eingesetzte Quenchkolonne;
- die in Schritt C) eingesetzten Kompressoren;
- die in Schritt C) eingesetzten Zwischenkühler;
- die in Schritt F) eingesetzte Destillationskolonne;
- der in der Lösungsmittelregeneriereinheit eingesetzte Dekanter.

3. Verfahren nach Anspruch 1 oder 2, **dadurch gekennzeichnet, dass** die in Schritt C) eingesetzten Zwischenkühler als Direktkühler ausgeführt sind.

4. Verfahren nach einem der Ansprüche 1 bis 3, **dadurch gekennzeichnet, dass** in den Schritten D), E) und/oder F) eingesetzte Destillationskolonnen als Bodenkolonnen mit gasdichten Kolonnenböden ausgeführt sind.

5. Verfahren nach einem der Ansprüche 1 bis 4, **dadurch gekennzeichnet, dass** Gaszuleitungen in die in den Schritten D), E) und/oder F) eingesetzten Destillationskolonnen in die Flüssigkeit eintauchen.

6. Verfahren nach einem der Ansprüche 1 bis 5, **dadurch gekennzeichnet, dass** Gasleitungen und gasführende Apparate ein derartiges Verhältnis Länge : Durchmesser aufweisen, dass eine Detonation ausgeschlossen ist.

7. Verfahren nach Anspruch 6, **dadurch gekennzeichnet, dass** das Verhältnis Länge : Durchmesser höchstens 50 : 1 beträgt.

8. Verfahren nach einem der Ansprüche 1 bis 7, **dadurch gekennzeichnet, dass** die Abkühlstufe Ca) zweistufig in zwei Abkühlstufen Ca1) und Ca2) durchgeführt wird, wobei beide Abkühlstufen als Direktkühler ausgebildet sind.

9. Verfahren nach Anspruch 8, **dadurch gekennzeichnet, dass** zumindest ein Teil des Kühlmittels nach Durchlaufen der zweiten Abkühlstufe Ca2) als Kühlmittel der ersten Abkühlstufe Ca1) zugeführt wird.

10. Verfahren nach einem der Ansprüche 8 und 9, **dadurch gekennzeichnet, dass** die Stufe Cb) im Allgemeinen mindestens eine Kompressionsstufe Cba) und mindestens eine Abkühlstufe Cbb) umfasst, wobei in der mindestens einen Abkühlstufe Cbb) das in der Kompressionsstufe Cba) komprimierte Gas mit einem Kühlmittel in Kontakt gebracht wird.

11. Verfahren nach Anspruch 10, **dadurch gekennzeichnet, dass** das Kühlmittel der Abkühlstufe Cbb) das gleiche organische Lösungsmittel, das in der Abkühlstufe Ca) als Kühlmittel verwendet wird, ist und zumindest ein Teil dieses Kühlmittels nach Durchlaufen der mindestens einen Abkühlstufe Cbb) als Kühlmittel der Abkühlstufe Ca) zugeführt wird.

12. Verfahren nach einem der Ansprüche 8 bis 11, **dadurch gekennzeichnet, dass** das im Kreis geführte Kühlmittel Mesitylen ist.

13. Verfahren nach einem der Ansprüche 1 bis 9, **dadurch gekennzeichnet, dass** Schritt D) die Schritte Da) bis Dc) umfasst:
Da) Absorption der C₄-Kohlenwasserstoffe umfassend Butadien und n-Butene in einem Absorptionsmittel, wobei ein mit C₄-Kohlenwasserstoffen beladener Absorptionsmittelstrom und der Gasstrom d2 erhalten werden,
Db) Entfernung von Sauerstoff aus dem mit C₄-Kohlenwasserstoffen beladenen Absorptionsmittelstrom aus Schritt Da) durch Strippung mit einem nicht kondensierbaren Gasstrom, und
Dc) Desorption der C₄-Kohlenwasserstoffe aus dem beladenen Absorptionsmittelstrom, wobei ein C₄-Produktgasstrom d1 erhalten wird.

14. Vorrichtung zur Durchführung des Verfahrens zur Herstellung von Butadien aus n-Butenen gemäß einem der Ansprüche 1 bis 13 umfassend:
A) Zuleitung zur Bereitstellung eines n-Butene enthaltenden Einsatzgasstroms a;
B) Zuleitungen zur Einspeisung des n-Butene enthaltenden Einsatzgasstromes a und eines sauerstoffhaltigen Gases in mindestens eine Dehydrierzone, Dehydrierzone umfassend einen Dehydrierreaktor zur oxidativen Dehydrierung von n-Butenen zu Butadien, wobei ein Produktgasstrom b enthaltend Butadien, nicht umgesetzte n-Butene, Wasserdampf, Sauerstoff, leicht siedende Kohlenwasserstoffe, gegebenenfalls Kohlenstoffoxide und gegebenenfalls Inertgase erhalten wird;
C) Zuleitung zur Einspeisung des Produktgasstroms b in eine Abkühlstufe und mindestens eine Abkühlstufe umfassend mindestens eine Quenchkolonne und mindestens eine Kompressionsstufe umfassend mindestens einen Kompressor und gegebenenfalls einen oder mehrere Zwischenkühler zwischen den Kompressoren, wobei der Produktgasstrom b mit mindestens einem im Kreis geführtem Kühlmittel in Kontakt gebracht wird, wobei mindestens ein Kondensatstrom c1 enthaltend Wasser und ein Gasstrom c2 enthaltend Butadien, n-Butene, Wasserdampf, Sauerstoff, leicht siedende Kohlenwasserstoffe, gegebenenfalls Kohlenstoffoxide und gegebenenfalls Inertgase erhalten wird;
D) Einrichtung zur Abtrennung von nicht kondensierbaren und leicht siedenden Gasbestandteilen umfassend Sauerstoff, leicht siedende Kohlenwasserstoffe, gegebenenfalls Kohlenstoffoxide und gegebenenfalls Inertgase als Gasstrom d2 aus dem Gasstrom c2 durch Absorption der C₄-Kohlenwasserstoffe umfassend Butadien und n-Butene in mindestens einem im Kreis geführten Absorptionsmittel, wobei mindestens ein mit C₄-Kohlenwasserstoffen beladener Absorptionsmittelstrom und der Gasstrom d2 erhalten werden, und Einrichtung zur anschließenden Desorption der C₄-Kohlenwasserstoffe aus dem beladenen Absorptionsmittelstrom, wobei ein C₄-Produktgasstrom d1 erhalten wird;
E) Extraktivdestillationskolonne zur Auftrennung des C₄-Produktstroms d1 durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2;
F) Destillationskolonne zur Destillation des Butadien und das selektive Lösungsmittel enthaltenden Stoffstroms e1 in einen im Wesentlichen aus dem selektiven Lösungsmittel bestehenden Stoffstrom f1 und einen Butadien enthaltenden Stoffstrom f2;
**dadurch gekennzeichnet, dass**
einer oder mehrere der zur Durchführung der Schritte A) bis F) eingesetzten Apparate explosionsfest ausgeführt sind, flüssigkeitsführende Rohrleitungen explosionsfest ausgeführt sind und Gasleitungen detonationsfest ausgeführt sind.

15. Vorrichtung nach Anspruch 14, **dadurch gekennzeichnet, dass** einer oder mehrere der nachstehenden Apparate explosionsfest ausgelegt sind:
- der in Schritt B) eingesetzte Dehydrierreaktor;
- die in Schritt C) eingesetzte Quenchkolonne;
- die in Schritt C) eingesetzten Kompressoren;
- die in Schritt C) eingesetzten Zwischenkühler;
- die in Schritt F) eingesetzte Destillationskolonne;
- der in der Lösungsmittelregeneriereinheit eingesetzte Dekanter.

## Claims

1. A process for preparing butadiene from n-butenes, which comprises the following steps:
A) provision of a feed gas stream a comprising n-butenes;
B) introduction of the feed gas stream a comprising n-butenes and an oxygen-comprising gas into at least one dehydrogenation zone comprising a dehydrogenation reactor and oxidative dehydrogenation of n-butenes to butadiene, giving a product gas stream b comprising butadiene, unreacted n-butenes, water vapor, oxygen, low-boiling hydrocarbons, possibly carbon oxides and possibly inert gases;
C) cooling and compression of the product gas stream b in at least one cooling stage comprising at least one quenching column and in at least one compression stage comprising at least one compressor and optionally one or more intermediate coolers between the compressors, with the product gas stream b being brought into contact with at least one circulated coolant to give at least one condensate stream c1 comprising water and a gas stream c2 comprising butadiene, n-butenes, water vapor, oxygen, low-boiling hydrocarbons, possibly carbon oxides and possibly inert gases;
D) separating off of incondensable and low-boiling gas constituents comprising oxygen, low-boiling hydrocarbons, possibly carbon oxides and possibly inert gases as gas stream d2 from the gas stream c2 by absorption of the C₄-hydrocarbons comprising butadiene and n-butenes in at least one circulated absorption medium, giving at least one absorption medium stream loaded with C₄-hydrocarbons and the gas stream d2, and subsequent desorption of the C₄-hydrocarbons from the loaded absorption medium stream to give a C₄ product gas stream d1 ;
E) separation of the C₄ product stream d1 by extractive distillation using a solvent which is selective for butadiene into a stream e1 comprising butadiene and the selective solvent and a stream e2 comprising n-butenes;
F) distillation of the stream e1 comprising butadiene and the selective solvent to give a stream f1 consisting essentially of the selective solvent and a stream f2 comprising butadiene;
wherein the measures (i) to (iii) below are carried out:
(i) avoidance of formation of explosive gas mixtures by monitoring of the oxygen concentration in the oxygen-comprising gas streams fed into the dehydrogenation zone and control of the mass flows of oxygen-comprising gas streams and gas streams comprising hydrocarbons in such a way that no explosive gas mixtures can be formed;
(ii) interruption of the introduction of the oxygen-comprising gas mixture into the dehydrogenation zone when a limit value for the oxygen concentration in the dehydrogenation gas mixture is exceeded;
(iii) carrying out of the steps A) to F) in apparatuses which are configured so as to be explosion-protected, where liquid-conveying pipes are configured so as to be explosion-protected and gas conduits are configured so as to be detonation-protected.

2. The process according to claim 1, wherein one or more of the following apparatuses are designed so as to be explosion-protected:
- the dehydrogenation reactor used in step B);
- the quenching column used in step C);
- the compressors used in step C);
- the intermediate coolers used in step C);
- the distillation column used in step F);
- the decanter used in the solvent regeneration unit.

3. The process according to claim 1 or 2, wherein the intermediate coolers used in step C) are configured as direct coolers.

4. The process according to any of claims 1 to 3, wherein distillation columns used in steps D), E) and/or F) are configured as tray columns having gastight column trays.

5. The process according to any of claims 1 to 4, wherein gas feed lines into the distillation columns used in steps D), E) and/or F) dip into the liquid.

6. The process according to any of claims 1 to 5, wherein gas conduits and gas-conveying apparatuses have such a ratio of length:diameter that detonation is ruled out.

7. The process according to claim 6, wherein the ratio of length:diameter is not more than 50:1.

8. The process according to any of claims 1 to 7, wherein the cooling stage Ca) is carried out in two cooling stages Ca1) and Ca2) and both cooling stages are designed as direct cooling units.

9. The process according to claim 8, wherein at least part of the coolant which has passed through the second cooling stage Ca2) is fed as coolant to the first cooling stage Ca1) .

10. The process according to either of claims 8 and 9, wherein the stage Cb) generally comprises at least one compression stage Cba) and at least one cooling stage Cbb), where the gas which has been compressed in the compression stage Cba) is brought into contact with a coolant in the at least one cooling stage Cbb).

11. The process according to claim 10, wherein the coolant of the cooling stage Cbb) is the same organic solvent which is used as coolant in the cooling stage Ca) and at least part of this coolant is, after passing through the at least one cooling stage Cbb), fed as coolant to the cooling stage Ca) .

12. The process according to any of claims 8 to 11, wherein the circulated coolant is mesitylene.

13. The process according to any of claims 1 to 9, wherein step D) comprises the steps Da) to Dc):
Da) absorption of the C₄-hydrocarbons comprising butadiene and n-butenes in an absorption medium to give an absorption medium stream loaded with C₄-hydrocarbons and the gas stream d2,
Db) removal of oxygen from the absorption medium stream loaded with C₄-hydrocarbons from step Da) by stripping with an incondensable gas stream and
Dc) desorption of the C₄-hydrocarbons from the loaded absorption medium stream to give a C₄ product gas stream d1.

14. An apparatus for carrying out the process for preparing butadiene from n-butenes according to any of claims 1 to 13, which comprises:
A) feed line for providing a feed gas stream a comprising n-butenes;
B) feed lines for introducing the feed gas stream a comprising n-butenes and an oxygen-comprising gas into at least one dehydrogenation zone, dehydrogenation zone comprising a dehydrogenation reactor for the oxidative dehydrogenation of n-butenes to butadiene, giving a product gas stream b comprising butadiene, unreacted n-butenes, water vapor, oxygen, low-boiling hydrocarbons, possibly carbon oxides and possibly inert gases;
C) feed line for introducing the product gas stream b into a cooling stage and at least one cooling stage comprising at least one quenching column and at least one compression stage comprising at least one compressor and optionally one or more intermediate coolers between the compressors, with the product gas stream b being brought into contact with at least one circulated coolant to give at least one condensate stream c1 comprising water and a gas stream c2 comprising butadiene, n-butenes, water vapor, oxygen, low-boiling hydrocarbons, possibly carbon oxides and possibly inert gases;
D) facility for separating off incondensable and low-boiling gas constituents comprising oxygen, low-boiling hydrocarbons, possibly carbon oxides and possibly inert gases as gas stream d2 from the gas stream c2 by absorption of the C₄-hydrocarbons comprising butadiene and n-butenes in at least one circulated absorption medium, giving at least one absorption medium stream loaded with C₄-hydrocarbons and the gas stream d2, and facility for subsequent desorption of the C₄-hydrocarbons from the loaded absorption medium stream to give a C₄ product gas stream d1 ;
E) extractive distillation column for separating the C₄ product stream d1 by extractive distillation using a solvent which is selective for butadiene into a stream e1 comprising butadiene and the selective solvent and a stream e2 comprising n-butenes;
F) distillation column for distillation of the stream e1 comprising butadiene and the selective solvent to give a stream f1 consisting essentially of the selective solvent and a stream f2 comprising butadiene;
wherein
one or more of the apparatuses used for carrying out steps A) to F) are configured so as to be explosion-protected, liquid-conveying pipes are configured so as to be explosion-protected and gas conduits are configured so as to be detonation-protected.

15. The apparatus according to claim 14, wherein one or more of the following apparatuses are designed so as to be explosion-protected:
- the dehydrogenation reactor used in step B);
- the quenching column used in step C);
- the compressors used in step C);
- the intermediate coolers used in step C);
- the distillation column used in step F);
- the decanter used in the solvent regeneration unit.

## Revendications

1. Procédé de fabrication de butadiène à partir de n-butènes, comprenant les étapes suivantes :
A) la préparation d'un courant gazeux d'entrée a contenant des n-butènes ;
B) l'introduction du courant gazeux d'entrée a contenant des n-butènes et d'un gaz contenant de l'oxygène dans au moins une zone de déshydrogénation comprenant un réacteur de déshydrogénation, et la déshydrogénation oxydative de n-butènes en butadiène, un courant gazeux de produits b contenant du butadiène, des n-butènes non réagis, de la vapeur d'eau, de l'oxygène, des hydrocarbures de point d'ébullition bas, éventuellement des oxydes de carbone et éventuellement des gaz inertes étant obtenu ;
C) le refroidissement et la compression du courant gazeux de produits b dans au moins une étape de refroidissement comprenant au moins une colonne de trempe et dans au moins une étape de compression comprenant au moins un compresseur et éventuellement un ou plusieurs refroidisseurs intermédiaires entre les compresseurs, le courant gazeux de produits b étant mis en contact avec au moins un agent réfrigérant en circulation dans un circuit, au moins un courant de condensat c1 contenant de l'eau et un courant gazeux c2 contenant du butadiène, des n-butènes, de la vapeur d'eau, de l'oxygène, des hydrocarbures de point d'ébullition bas, éventuellement des oxydes de carbone et éventuellement des gaz inertes étant obtenus ;
D) la séparation de constituants gazeux non condensables et de point d'ébullition bas, comprenant de l'oxygène, des hydrocarbures de point d'ébullition bas, éventuellement des oxydes de carbone et éventuellement des gaz inertes, en tant que courant gazeux d2 à partir du courant gazeux c2 par absorption des hydrocarbures en C₄ comprenant le butadiène et les n-butènes dans au moins un agent d'absorption mis en circulation dans un circuit, au moins un courant d'agent d'absorption chargé avec des hydrocarbures en C₄ et le courant gazeux d2 étant obtenus, puis la désorption des hydrocarbures en C₄ à partir du courant d'agent d'absorption chargé, un courant gazeux de produits en C₄ d1 étant obtenu ;
E) la séparation du courant de produits en C₄ d1 par distillation extractive avec un solvant sélectif pour le butadiène en un courant de matière e1 contenant le butadiène et le solvant sélectif, et un courant de matière e2 contenant les n-butènes ;
F) la distillation du courant de matière e1 contenant le butadiène et le solvant sélectif en un courant de matière f1 essentiellement constitué par le solvant sélectif et un courant de matière f2 contenant le butadiène ;
**caractérisé en ce que** les mesures (i) à (iii) suivantes sont réalisées :
(i) l'évitement de la formation de mélanges gazeux explosifs par surveillance de la concentration en oxygène dans les courants gazeux contenant de l'oxygène introduits dans la zone de déshydrogénation et ajustement des débits massiques des courants gazeux contenant de l'oxygène et des courants gazeux contenant des hydrocarbures de telle sorte que des mélanges gazeux explosifs ne puissent pas se former ;
(ii) l'interruption de l'introduction du mélange gazeux contenant de l'oxygène dans la zone de déshydrogénation lors du dépassement d'une valeur limite pour la concentration en oxygène dans le mélange gazeux de déshydrogénation ;
(iii) la réalisation des étapes A) à F) dans des appareils qui sont configurés sous une forme résistante aux explosions, les canalisations de conduction de liquides étant configurées sous une forme résistante aux explosions et les conduites de gaz étant configurées sous une forme résistante aux détonations.

2. Procédé selon la revendication 1, **caractérisé en ce qu'**un ou plusieurs des appareils suivants sont configurés sous une forme résistante aux explosions :
- le réacteur de déshydrogénation utilisé à l'étape B) ;
- la colonne de trempe utilisée à l'étape C) ;
- les compresseurs utilisés à l'étape C) ;
- les refroidisseurs intermédiaires utilisés à l'étape C) ;
- la colonne de distillation utilisée à l'étape F) ;
- le décanteur utilisé dans l'unité de régénération du solvant.

3. Procédé selon la revendication 1 ou 2, **caractérisé en ce que** les refroidisseurs intermédiaires utilisés à l'étape C) sont configurés sous la forme de refroidisseurs directs.

4. Procédé selon l'une quelconque des revendications 1 à 3, **caractérisé en ce que** les colonnes de distillation utilisées dans les étapes D), E) et/ou F) sont configurées sous la forme de colonnes à plateaux munies de plateaux de colonne étanches aux gaz.

5. Procédé selon l'une quelconque des revendications 1 à 4, **caractérisé en ce que** les conduites d'alimentation de gaz dans les colonnes de distillation utilisées dans les étapes D), E) et/ou F) sont immergées dans le liquide.

6. Procédé selon l'une quelconque des revendications 1 à 5, **caractérisé en ce que** les conduites de gaz et les appareils conducteurs de gaz présentent un rapport longueur:diamètre tel qu'une détonation soit exclue.

7. Procédé selon la revendication 6, **caractérisé en ce que** le rapport longueur:diamètre est d'au plus 50:1.

8. Procédé selon l'une quelconque des revendications 1 à 7, **caractérisé en ce que** l'étape de refroidissement Ca) est réalisée en deux étapes par deux étapes de refroidissement Ca1) et Ca2), les deux étapes de refroidissement étant configurées sous la forme de refroidisseurs directs.

9. Procédé selon la revendication 8, **caractérisé en ce qu'**au moins une partie de l'agent réfrigérant est introduite en tant qu'agent réfrigérant de la première étape de refroidissement Ca1) après la traversée de la deuxième étape de refroidissement Ca2).

10. Procédé selon l'une quelconque des revendications 8 et 9, **caractérisé en ce que** l'étape Cb) comprend généralement au moins une étape de compression Cba) et au moins une étape de refroidissement Cbb), le gaz comprimé dans l'étape de compression Cba) étant mis en contact avec un agent réfrigérant lors de ladite au moins une étape de refroidissement Cbb).

11. Procédé selon la revendication 10, **caractérisé en ce que** l'agent réfrigérant de l'étape de refroidissement Cbb) est le même solvant organique que celui utilisé dans l'étape de refroidissement Ca) en tant qu'agent réfrigérant, et au moins une partie de cet agent réfrigérant est introduite en tant qu'agent réfrigérant de l'étape de refroidissement Ca) après la traversée de ladite au moins une étape de refroidissement Cbb).

12. Procédé selon l'une quelconque des revendications 8 à 11, **caractérisé en ce que** l'agent réfrigérant mis en circulation dans le circuit est le mésitylène.

13. Procédé selon l'une quelconque des revendications 1 à 9, **caractérisé en ce que** l'étape D) comprend les étapes Da) à Dc) :
Da) l'absorption des hydrocarbures en C₄ comprenant le butadiène et les n-butènes dans un agent d'absorption,
un courant d'agent d'absorption chargé avec des hydrocarbures en C₄ et le courant gazeux d2 étant obtenus ;
Db) l'élimination de l'oxygène du courant d'agent d'absorption chargé avec des hydrocarbures en C₄ de l'étape Da) par extraction avec un courant gazeux non condensable ; et
Dc) la désorption des hydrocarbures en C₄ du courant d'agent d'absorption chargé, un courant gazeux de produits en C₄ d1 étant obtenu.

14. Dispositif pour la réalisation du procédé de fabrication de butadiène à partir de n-butènes selon l'une quelconque des revendications 1 à 13, comprenant :
A) une conduite d'alimentation pour la mise à disposition d'un courant gazeux d'entrée a contenant des n-butènes ;
B) des conduites d'alimentation pour l'introduction du courant gazeux d'entrée a contenant des n-butènes et d'un gaz contenant de l'oxygène dans au moins une zone de déshydrogénation, une zone de déshydrogénation comprenant un réacteur de déshydrogénation pour la déshydrogénation oxydative de n-butènes en butadiène, un courant gazeux de produits b contenant du butadiène, des n-butènes non réagis, de la vapeur d'eau, de l'oxygène, des hydrocarbures de point d'ébullition bas, éventuellement des oxydes de carbone et éventuellement des gaz inertes étant obtenu ;
C) une conduite d'alimentation pour l'introduction du courant gazeux de produits b dans une étape de refroidissement, et au moins une étape de refroidissement comprenant au moins une colonne de trempe et au moins une étape de compression comprenant au moins un compresseur et éventuellement un ou plusieurs refroidisseurs intermédiaires entre les compresseurs, le courant gazeux de produits b étant mis en contact avec au moins un agent réfrigérant en circulation dans un circuit, au moins un courant de condensat c1 contenant de l'eau et un courant gazeux c2 contenant du butadiène, des n-butènes, de la vapeur d'eau, de l'oxygène, des hydrocarbures de point d'ébullition bas, éventuellement des oxydes de carbone et éventuellement des gaz inertes étant obtenus ;
D) un dispositif pour la séparation de constituants gazeux non condensables et de point d'ébullition bas, comprenant de l'oxygène, des hydrocarbures de point d'ébullition bas, éventuellement des oxydes de carbone et éventuellement des gaz inertes, en tant que courant gazeux d2 à partir du courant gazeux c2 par absorption des hydrocarbures en C₄ comprenant le butadiène et les n-butènes dans au moins un agent d'absorption mis en circulation dans un circuit, au moins un courant d'agent d'absorption chargé avec des hydrocarbures en C₄ et le courant gazeux d2 étant obtenus, et un dispositif pour la désorption ultérieure des hydrocarbures en C₄ à partir du courant d'agent d'absorption chargé, un courant gazeux de produits en C₄ d1 étant obtenu ;
E) une colonne de distillation extractive pour la séparation du courant de produits en C₄ d1 par distillation extractive avec un solvant sélectif pour le butadiène en un courant de matière e1 contenant le butadiène et le solvant sélectif, et un courant de matière e2 contenant les n-butènes ;
F) une colonne de distillation pour la distillation du courant de matière e1 contenant le butadiène et le solvant sélectif en un courant de matière f1 essentiellement constitué par le solvant sélectif et un courant de matière f2 contenant le butadiène ;
**caractérisé en ce qu'**un ou plusieurs des appareils utilisés pour la réalisation des étapes A) à F) sont configurés sous une forme résistante aux explosions, les canalisations de conduction de liquides sont configurées sous une forme résistante aux explosions et les conduites de gaz sont configurées sous une forme résistante aux détonations.

15. Dispositif selon la revendication 14, **caractérisé en ce qu'**un ou plusieurs des appareils suivants sont configurés sous une forme résistante aux explosions :
- le réacteur de déshydrogénation utilisé à l'étape B) ;
- la colonne de trempe utilisée à l'étape C) ;
- les compresseurs utilisés à l'étape C) ;
- les refroidisseurs intermédiaires utilisés à l'étape C) ;
- la colonne de distillation utilisée à l'étape F) ;
- le décanteur utilisé dans l'unité de régénération du solvant.
